# FASCICULE DE BREVET EUROPEEN

(11) **EP 3 160 424 B1**
(45) Date de publication et mention de la délivrance du brevet: **09.12.2020**
(21) Numéro de dépôt: 15745530.4
(22) Date de dépôt: 30.06.2015
(51) Int. Cl.: A61K 8/25, A61K 8/37, A61K 8/44, A61Q 1/00, A61K 8/73

(54) **COMPOSITION ANHYDRE COMPRENANT UN GÉLIFIANT LIPOPHILE, AU MOINS UNE CHARGE PARTICULIÈRE ET UNE PHASE GRASSE**
WASSERFREIE ZUSAMMENSETZUNG ENTHALTEND EIN LIPOPHILES GELIERUNGSMITTEL, EINE BESONDERE LADUNG UND EINE FETTPHASE
ANHYDROUS COMPOSITION COMPRISING A LIPOPHILIC GELLING AGENT, AT LEAST A SPECIFIC FILLER AND A FATTY PHASE

(30) Priorité: 30.06.2014 FR 1456211
(43) Date de publication de la demande: 03.05.2017
(73) Titulaire: L'OREAL, 75008 Paris (FR)
(72) Inventeur: PAGE, Valérie, F-26400 Saoû (FR); GUILBAUD, Sophie, F-26270 Cliousclat (FR); DEMARCQ, Céline, F-26400 Gigors et Lozeron (FR)
(74) Mandataire: L'Oreal
(86) Numéro de dépôt international: PCT/FR2015/051799
(87) Numéro de publication internationale: WO 2016/001577

(56) Documents cités:
- EP-A1- 1 386 600
- FR-A1- 2 843 021
- FR-A1- 2 954 103
- FR-A1- 2 975 297

## Description

La présente invention concerne une composition anhydre comprenant de 3 % à 15 % en poids d'au moins un gélifiant lipophile, de 20 % à 50 % en poids d'au moins une charge choisie parmi les particules sphériques de silice poreuse, les particules sphériques de cellulose, et les poudres d'acide aminé N-acylé ayant un groupe acyle en C8-C22, et de 40 % à 85 % en poids d'au moins une phase grasse contenant au moins 20 % en poids d'au moins une huile choisie parmi les huiles hydrocarbonées d'origine végétale, de préférence les triglycérides, les alcools de Guerbet et les esters d'acide gras en C8-C30 et d'alcool de Guerbet, les éthers d'alcools gras comportant de 6 à 20 atomes de carbone. L'invention concerne en particulier le domaine cosmétique, et notamment le domaine du soin et/ou du maquillage des matières kératiniques, notamment de la peau, des lèvres, des cheveux ou des ongles, de préférence la peau.

Dans le domaine des compositions cosmétiques de soin et/ou de maquillage de la peau, il est connu d'utiliser des charges minérales ou organiques à effet flouteur « soft-focus » qui absorbe le gras, assure un effet optique bluffant « photoshop », ainsi qu'un toucher tout doux, pour matifier la peau, et/ou lisser optiquement le microrelief, combler les rides, camoufler les imperfections de la peau et mieux réfléchir la lumière.

Toutefois, l'utilisation de ces charges s'accompagne en général d'un toucher sec, rèche, de peluches, de traces blanches et d'un manque de confort rhédibitoire pour l'utilisatrice.

Afin de remédier à ce problème et disposer d'un soin confortable ayant un effet flouteur, des galéniques huileuses sont actuellement proposées sur le marché cosmétique. Cependant, elles présentent les inconvénients liés à la présence de forts taux de phase grasse dans la composition, à savoir l'obtention d'une peau luisante et/ou la sensation de peau grasse et/ou collante.

On connaît aussi pour répondre à cette problématique l'utilisation de silicones réticulées. Ce type de matières premières permet en effet de combiner effet mat et soft-focus, mais présente l'inconvénient d'être caractérisé par un toucher gras et chaud peu confortable, avec un effet « masque ».

Ainsi, il est difficile de concilier dans une même composition des performances techniques qui s'opposent telles que par exemple matité (qui peut rendre la peau sèche) et hydratation (qui peut rendre la peau brillante).

Il demeure ainsi difficile pour l'homme de l'art de proposer des compositions homogènes aptes à procurer un résultat visuel immédiat sur la peau avec une sensation de légèreté et de confort à l'application, ce résultat immédiat attendu étant préférentiellement une bonne couvrance des imperfections colorielles et/ou des imperfections de reliefs.

Il subsiste donc le besoin de préparer des compositions cosmétiques résolvant le problème technique de matifier et/ou lisser optiquement le microrelief de la peau, de combler les rides, camoufler les imperfections de la peau et mieux réfléchir la lumière tout en procurant un toucher agréable notamment lors de son application, un fini peau tout doux sans effet « masque » et qui laisse la peau respirer.

La demanderesse a découvert que ce besoin pouvait être satisfait en associant, dans une composition anhydre comprenant une phase grasse convenablement sélectionnée, un gélifiant lipophile et des charges convenablement sélectionnées, dans des quantités appropriées.

Cette association permet de répondre à la problématique citée ci-dessus en proposant une galénique huileuse, forte d'une sensorialité nouvelle tant à l'application que sur le fini peau sans négliger les qualités cosmétiques.

Plus précisément, la présente invention a pour objet une composition anhydre comprenant :
- de 3 % à 15 % en poids d'au moins un gélifiant lipophile ;
- de 20 % à 50 % en poids d'au moins une charge choisie parmi les particules sphériques de silice poreuse, les particules sphériques de cellulose, et les poudres d'acide aminé N-acylé ayant un groupe acyle en C8-C22 ; et
- de 40 % à 85 % en poids d'au moins une phase grasse contenant au moins 20 % en poids d'au moins une huile choisie parmi les huiles hydrocarbonées d'origine végétale, de préférence les triglycérides, les alcools de Guerbet et les esters d'acide gras en C8-C30 et d'alcool de Guerbet, les éthers d'alcools gras comportant de 6 à 20 atomes de carbone;

les quantités en poids étant données par rapport au poids total de la composition.

Selon un mode de réalisation particulier de l'invention, la composition anhydre comprend :
- de 3 % à 15 % en poids d'au moins un gélifiant lipophile ;
- de 20 % à 50 % en poids d'au moins une charge choisie parmi les particules sphériques de silice poreuse, les particules sphériques de cellulose, et les poudres d'acide aminé N-acylé ayant un groupe acyle en C8-C22 ; et
- de 40 % à 85 % en poids d'au moins une phase grasse contenant au moins 25 % en poids d'au moins une huile choisie parmi les huiles hydrocarbonées d'origine végétale, de préférence les triglycérides, les alcools de Guerbet et les esters d'acide gras en C8-C30 et d'alcool de Guerbet;

les quantités en poids étant données par rapport au poids total de la composition.

La présente invention permet d'obtenir une nouvelle galénique tout particulièrement intéressante au regard de ses performances techniques et des ressentis sensoriels qu'elle procure à l'utilisateur lors de son application sur les matières kératiniques, et en particulier sur la peau.

Ainsi, la composition conforme à l'invention permet de disposer d'un soin confortable anhydre à effet flouteur « soft-focus », c'est-à-dire permettant de matifier et/ou lisser optiquement le microrelief de la peau, combler les rides, camoufler les imperfections de la peau, mieux réfléchir la lumière. Elle apporte un effet optique et tactile pour corriger en surface et immédiatement l'aspect de la peau, en atténuant les zones d'ombre du relief cutané tels que les rides, les ridules, les pores dilatés et autres imperfections cutanées. Elle estompe les signes de fatigue. Elle lisse et affine le grain de la peau, lui redonne éclat et fraicheur, pour un résultat naturel.

La composition répond au besoin des consommatrices d'avoir à disposition un produit huileux sans en avoir les inconvénients d'application et de fini peau engendrés par un type de galénique huileuse classique, à savoir sans avoir le toucher gras ni l'aspect luisant, peu ou pas collant. La composition pénètre bien lors de son application sur la peau.

Le fini peau peut être poudré, velouté, homogène et confortable.

Le fini peau peut également être un fini filmogène avantageusement gainant, peu ou pas collant.

Le confort à l'application se traduit notamment par une absence de tiraillements, de sensations de dessèchement.

La composition selon l'invention est stable, notamment dans le temps et/ou aux variations de température, tout en présentant de bonnes propriétés cosmétiques et sensorielles.

Par « stable », on entend stable à température ambiante (25 °C) au moins un mois, de préférence au moins 2 mois.

La présente invention a également pour objet un procédé cosmétique de maquillage et/ou de soin des matières kératiniques comprenant une étape d'application d'une composition telle que définie plus haut sur lesdites matières kératiniques.

La composition de l'invention étant destinée à une application topique sur la peau ou les phanères, elle comprend un milieu physiologiquement acceptable, c'est-à-dire un milieu compatible avec toutes les matières kératiniques telles que la peau, les ongles, les muqueuses et les fibres kératiniques (telles que les cheveux, les cils).

Par « anhydre », au sens de la présente invention, on entend une composition comprenant une teneur inférieure ou égale à 1 % en poids d'eau, de préférence inférieure ou égale à 0,5 % en poids par rapport au poids total de ladite composition, voire exempte d'eau. Le cas échéant, d'aussi faibles quantités d'eau peuvent notamment être amenées par des ingrédients de la composition qui peuvent en contenir des quantités résiduelles.

Dans ce qui va suivre, l'expression « au moins un(e) » est équivalente à « un(e) ou plusieurs » et, à moins d'une autre indication, les bornes d'un domaine de valeurs sont comprises dans ce domaine.

### Gélifiants lipophiles

On entend par « gélifiant lipophile », au sens de la présente invention, un composé apte à gélifier la phase grasse de la composition selon l'invention.

Le ou les gélifiants lipophiles sont liposolubles ou lipodispersibles.

Selon un mode de réalisation particulier de l'invention, le ou les gélifiants lipophiles sont organiques ou minéraux.

Selon un autre mode de réalisation particulier de l'invention, le ou les gélifiants lipophiles sont non siliconés.

Le ou les gélifiants utilisables dans le cadre de l'invention peuvent être des gélifiants lipophiles organiques, polymériques ou moléculaires.

A titre d'exemples de gélifiants lipophiles pouvant être utilisés dans le cadre de l'invention, on peut citer les micas naturels modifiés tel que le fluorosilicate d'aluminium, de magnésium et de potassium, en particulier celui qui est commercialisé par la société SENSIENT sous la dénomination SUBMICA M, les esters de dextrine et d'acide gras, notamment en C7-C51, tels que le palmitate de dextrine, en particulier celui qui est commercialisé par la société CHIBA FLOUR MILLING sous la dénomination RHEOPEARL TL2-OR, ou le palmitate de dextrine commercialisé par la même société sous la dénomination RHEOPEARL KL2-OR, ainsi que le myristate de dextrine, en particulier celui qui est commercialisé par la société CHIBA FLOUR MILLING sous la dénomination RHEOPEARL MKL2, les tri-esters d'acide gras en C8-C30 et de mono ou polyglycérol tels que le tri-hydroxy-stéarate de glycéryle, en particulier celui qui est commercialisé par la société ELEMENTIS sous la dénomination THIXCIN R. On pourra aussi mentionner le RHEOCIN commercialisé par la société BYK ADDITIVES & INSTRUMENTS.

Selon un mode de réalisation particulier de l'invention, le ou les gélifiants lipophiles sont organiques.

Selon un mode de réalisation particulier, le ou les gélifiants lipophiles utilisables dans le cadre de l'invention sont choisis parmi les esters de dextrine et d'acide gras et les tri-esters d'acide gras en C8-C30 et de mono ou polyglycérol.

### Esters de dextrine et d'acide gras

Les esters de dextrine et d'acide gras utilisés selon l'invention peuvent être notamment choisis parmi les mono- ou poly-ester de dextrine et d'au moins un acide gras répondant à la formule (C) : 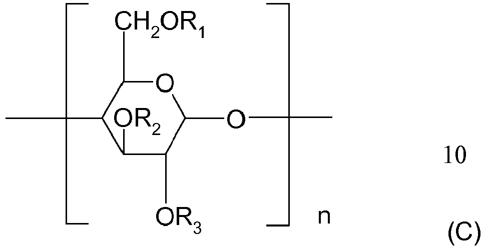 dans laquelle :
n est un entier allant de 3 à 150, notamment de 10 à 100, et de préférence de 15 à 40 ;
les radicaux R₁, R₂ et R₃ représentent un atome d'hydrogène ou un groupement acyle (R-CO-) dans lequel le radical R est un groupement hydrocarboné, linéaire ou ramifié, saturé ou insaturé, possédant 6 à 50, notamment 8 à 30, voire 12 à 22, et mieux 12 à 18 atomes de carbone, sous réserve qu'au moins un desdits radicaux R₁, R₂ et R₃ est différent d'un atome d'hydrogène.

En particulier, R₁, R₂ et R₃ peuvent représenter l'hydrogène ou un groupement acyle (R-CO-) dans lequel R est un radical hydrocarboné tel que défini précédemment, sous réserve qu'au moins deux desdits radicaux R₁, R₂ ou R₃ sont identiques et différents de l'hydrogène. L'ensemble des radicaux R₁, R₂ et R₃ peuvent figurer un groupement acyle (R-CO) identique ou différent et notamment identique.

Le radical R-CO- de l'ester de dextrine de formule (C) peut notamment être choisi parmi les radicaux caprylyle, capryle, lauryle, myristyle, palmityle, stéaryle, eicosanyle, docosanyle, isovaléryle, éthyl-2 butyryle, éthylméthylacétyle, isoheptanyle, éthyl-2 hexanyle, isononanyle, isodécanyle, isotridécanyle, isomyristyle, isopalmityle, isostéaryle, isohexanyle, décènyle, dodécenyle, tétradécényle, myristyle, hexadécényle, palmitoléyle, oléyle, élaidyle, eicosényle, sorbyle, linoléyle, linolényle, punicyle, arachidonyle, stéarolyle, et leurs mélanges.

Le radical R-CO est avantageusement linéaire. R-CO est de préférence le radical palmityle ou le radical myristyle, et encore plus préférentiellement le radical palmityle.

n va avantageusement de 25 à 35, de préférence va de 27 à 33, et mieux est égal à 30.

De préférence, on utilise à titre d'ester de dextrine et d'acide(s) gras au moins un palmitate de dextrine et/ou au moins un myristate de dextrine. Ceux-ci peuvent être utilisé seuls ou en mélange avec d'autres esters.

Avantageusement, l'ester de dextrine et d'acide gras a un degré de substitution inférieur ou égal à 2,5 sur la base d'une unité glucose, notamment variant de 1,5 à 2,5. Le poids moléculaire moyen en poids de l'ester de dextrine peut être en particulier de 10 000 à 150 000, notamment de 12 000 à 100 000 et voire de 15 000 à 80 000.

Des esters de dextrine sont disponibles commercialement, en particulier des palmitates de dextrine, par exemple sous la dénomination RHEOPEARL TL2-OR ou RHEOPEARL KL2-OR de la société CHIBA FLOUR MILLING, et sous la dénomination RHEOPEARL KS de la société CHIBA FLOUR MILLING, et des myristates de dextrine, par exemple sous la dénomination RHEOPEARL MKL2 de la société CHIBA FLOUR MILLING.

Selon un mode de réalisation particulier de l'invention, on utilisera un mélange d'un ester de dextrine et d'acide gras dont le degré de substitution est inférieur à 2 sur la base d'une unité glucose et d'un ester de dextrine et d'acide gras dont le degré de substitution est supérieur à 2 sur la base d'une unité glucose, comme décrit dans la demande FR 2 843 020.

Selon un mode de mise en oeuvre, l'ester de dextrine et d'acide gras dont le degré de substitution est inférieur à 2 sur la base d'une unité glucose répond avantageusement à la formule (IV) suivante : 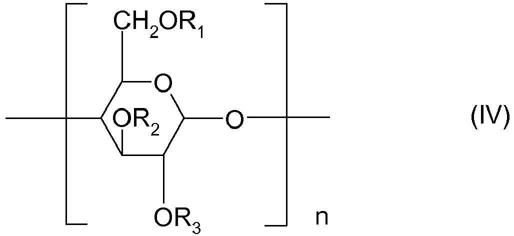 dans laquelle :
les radicaux R₁, R₂ et R₃ représentent un atome d'hydrogène ou un groupement acyle (R-CO-) dans lequel le radical R est un groupement hydrocarboné, linéaire ou ramifié, saturé ou insaturé, possédant 6 à 50, notamment 8 à 30, voire 12 à 22 et mieux 12 à 18 atomes de carbone, sous réserve qu'au moins un desdits radicaux R₁, R₂ et R₃ est différent d'un atome d'hydrogène ;
n est un entier allant de 3 à 150, notamment de 10 à 100, et de préférence de 15 à 40.

Le radical R-CO- de l'ester de dextrine de formule (IV) peut notamment être choisi parmi les radicaux caprylyle, caproyle, lauroyle, myristyle, palmityle, stéaryle, eicosanyle, docosanoyle, isovaléryle, éthyl-2 butyryle, éthylméthylacétyle, isoheptanyle, éthyl-2 hexanyle, isononanyle, isodécanyle, isotridécanyle, isomyristyle, isopalmityle, isostéaryle, isohexanyle, décènyle, dodécenyle, tétradécényle, myristyle, hexadécénoyle, palmitoléyle, oléyle, élaidyle, eicosényle, sorbyle, linoléyle, linolényle, punicyle, arachidonyle, stéarolyle, et leurs mélanges.

Le radical R-CO est avantageusement linéaire. Le radical R-CO est de préférence le radical palmityle ou le radical myristyle, et encore plus préférentiellement le radical palmityle.

n va avantageusement de 25 à 35, de préférence va de 27 à 33, et mieux est égal à 30.

De préférence, on utilise un ester de dextrine d'acide gras dont le degré de substitution est inférieur à 2 sur la base d'une unité glucose, tel que le degré de substitution est inférieur à 1,9, de préférence inférieur à 1,8, de préférence encore est compris entre 1,5 et 1,7. Certains de ces esters de dextrine sont disponibles commercialement, notamment sous la dénomination RHEOPEARL TL de la société Chiba Flour Milling.

Le poids moléculaire moyen en poids de l'ester de dextrine et d'acide gras dont le degré de substitution est inférieur à 2 sur la base d'une unité glucose est de préférence compris entre 10.000 et 30.000, de préférence encore entre 15.000 et 20.000. Le poids moléculaire moyen en poids est déterminé en chromatographie en phase gazeuse, avec un étalonnage polystyrène.

Selon un mode de mise en oeuvre, l'ester de dextrine et d'acide gras dont le degré de substitution est supérieur à 2 sur la base d'une unité glucose répond à la formule (V) : 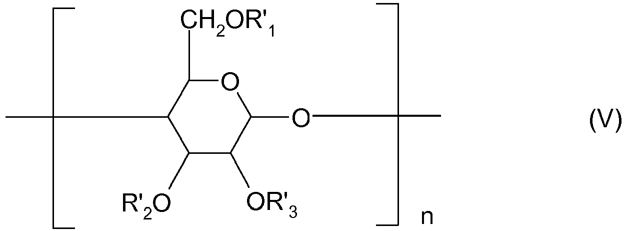 dans laquelle :
les radicaux R'₁, R'₂ et R'₃, identiques ou différents, sont choisis parmi un atome d'hydrogène ou un groupement acyle (R'-CO-) dans lequel le radical R' est un groupement hydrocarboné, linéaire ou ramifié, saturé ou insaturé, possédant 6 à 50, notamment 8 à 30, voire 12 à 22 et mieux 12 à 18 atomes de carbone, sous réserve qu'au moins un desdits radicaux R'₁, R'₂ et R'₃ est différent d'un atome d'hydrogène,
n est un entier allant de 3 à 150, notamment de 10 à 100, et de préférence de 15 à 40.
R' et n peuvent avoir la même signification que R et n décrits précédemment. Avantageusement, les radicaux R'₁, R'₂ et R'₃ sont identiques, sous réserve qu'au moins un desdits radicaux R'₁, R'₂ et R'₃ est différent d'un atome d'hydrogène.

De préférence, on utilise un ester de dextrine d'acide gras dont le degré de substitution est supérieur à 2 sur la base d'une unité glucose, tel que le degré de substitution est supérieur à 2,1, de préférence compris entre 2,1 et 2,3.

Le poids moléculaire moyen en poids de l'ester de dextrine et d'acide gras dont le degré de substitution est supérieur à 2 sur la base d'une unité glucose est de préférence compris entre 10.000 et 30.000, de préférence encore entre 15.000 et 20.000. Le poids moléculaire moyen en poids est déterminé en chromatographie en phase gazeuse, avec un étalonnage polystyrène.

A titre d'exemples d'esters de dextrine de formule (V) selon l'invention, on peut citer le Rheopearl KL vendu par la société CHIBA FLOUR MILLING.

### Tri-esters d'acide gras et de mono ou polyglycérol

Selon un mode de réalisation particulier, le ou les tri-esters d'acide gras et de mono ou polyglycérol sont des tri-esters d'acide gras et de monoglycérol.

Par acide gras, on entend un acide linéaire ou ramifié, saturé ou insaturé, comprenant de 8 à 30 atomes de carbone, de préférence de 10 à 24 atomes de carbone, encore plus préférentiellement de 12 à 22, et mieux de 16 à 20 atomes de carbone, substitué ou non substitué par un ou plusieurs groupements hydroxyle. Selon un mode de réalisation particulier de l'invention, le ou les acides gras sont linéaires, saturés, substitués par au moins un groupement hydroxyle.

Les acides gras peuvent être choisis parmi l'acide oléique, l'acide laurique, l'acide palmitique, l'acide myristique, l'acide stéarique, l'acide linoléique, l'acide caprique, l'acide béhénique, substitués ou non par au moins un groupement hydroxyle, ou leurs mélanges. De préférence, le ou les acides gras sont choisis parmi l'acide stéarique, les acides stéariques substitués par au moins un groupement hydroxyle, et leurs mélanges, plus préférentiellement ils sont choisis parmi l'acide stéarique, l'acide 12-hydroxy stéarique, et leurs mélanges, et encore mieux, il s'agit de l'acide 12-hydroxy stéarique.

Selon un mode de réalisation particulier de l'invention, le tri-ester d'acide gras en C8-C30 et de mono ou polyglycéryle est le tris(12-hydroxystéarate) de glycéryle.

A titre d'exemples de tri-ester d'acide gras en C8-C30 et de mono ou polyglycérol, on peut citer le tri-(hydroxystérate) de glycéryle (nom INCI: TRIHYDROXYSTEARIN) comme par exemple celui qui est commercialisé par la société ELEMENTIS sous la dénomination THIXCIN R ou celui qui est commercialisé par la société BYK ADDITIVES & INSTRUMENTS sous la dénomination RHEOCIN.

Selon un mode de réalisation particulier de l'invention, le ou les gélifiants lipophiles utilisables dans le cadre de l'invention sont choisis parmi les esters de dextrine et d'acide gras, de préférence le palmitate de dextrine et le myristate de dextrine.

Selon un autre mode de réalisation particulier de l'invention, le ou les gélifiants lipophiles utilisables dans le cadre de l'invention sont choisis parmi les tri-esters d'acide gras en C8-C30 et de monoglycérol, de préférence les tri(hydroxystéarate) de glycéryle, et encore plus préférentiellement le tris(12-hydroxystéarate) de glycéryle.

Le ou les gélifiants lipophiles sont présents dans la composition conforme à l'invention en quantité comprise entre 3 % et 15 % en poids du poids total de la composition, de préférence de 5 % à 12 % en poids, et encore plus préférentiellement de 8,5 % à 10 % en poids.

### Charges

La composition conforme à l'invention comprend de 20 % à 50 % en poids par rapport au poids total de la composition d'au moins une charge choisie parmi les particules sphériques de silice poreuse, les particules sphériques de cellulose, et les poudres d'acide aminé N-acylé ayant un groupe acyle en C8-C22.

Par charge, il faut comprendre des particules de toute forme, incolores ou blanches, minérales ou de synthèse, insolubles dans le milieu de la composition quelle que soit la température à laquelle la composition est fabriquée.

Les charges utilisées dans la présente invention peuvent être caractérisées par leur surface spécifique par unité de masse ou par unité de volume, leur taille exprimée en diamètre moyen en volume D(4,3), leur densité non tassée, et/ou leur capacité d'absorption d'huile. La surface spécifique par unité de masse peut être déterminée par la méthode d'absorption d'azote appelée méthode BET (BRUNAUER - EMMET - TELLER) décrite dans « The journal of the American Chemical Society », vol. 60, page 309, février 1938 et correspondant à la norme internationale ISO 5794/1 (annexe D). La surface spécifique BET correspond à la surface spécifique totale des particules considérées.

La surface spécifique par unité de volume est donnée par la relation : S_{V} = S_{M} x ρ ; où ρ est la densité tassée exprimée en g/cm3 et S_{M} est la surface spécifique par unité de masse exprimée en m²/g, telles que définie plus haut.

Dans le cadre de la présente invention, cette densité peut être appréciée selon le protocole suivant, dit de la densité tassée :
On verse 40 g de poudre dans une éprouvette graduée; puis on place l'éprouvette sur l'appareil STAV 2003 de chez STAMPF VOLUMETER ; l'éprouvette est ensuite soumise à une série de 2500 tassements (cette opération est recommencée jusqu'à ce que la différence de volume entre 2 essais consécutifs soit inférieure à 2%); puis on mesure directement sur l'éprouvette le volume final Vf de poudre tassée. La densité tassée est déterminée par le rapport m/Vf, en l'occurrence 40/Vf (Vf étant exprimé en cm³ et m en g).

La capacité d'absorption d'huile mesurée au Wet Point, et notée Wp, correspond à la quantité d'huile qu'il faut additionner à 100 g de particules pour obtenir une pâte homogène.

Elle est mesurée selon la méthode dite de Wet Point ou méthode de détermination de prise d'huile de poudre décrite dans la norme NF T 30-022. Elle correspond à la quantité d'huile adsorbée sur la surface disponible de la poudre et/ou absorbée par la poudre par mesure du Wet Point, décrite ci-dessous :
On place une quantité m= 2 g de poudre sur une plaque de verre puis on ajoute goutte à goutte l'huile (isononyl isononanoate). Après addition de 4 à 5 gouttes d'huile dans la poudre, on mélange à l'aide d'une spatule et on continue d'ajouter de l'huile jusqu'à la formation de conglomérats d'huile et de poudre. A partir de ce moment, on ajoute l'huile à raison d'une goutte à la fois et on triture ensuite le mélange avec la spatule. On cesse l'addition d'huile lorsque l'on obtient une pâte ferme et lisse. Cette pâte doit se laisser étendre sur la plaque de verre sans craquelures ni formation de grumeaux. On note alors le volume Vs (exprimé en ml) d'huile utilisé.

La prise d'huile correspond au rapport Vs / m.

Selon un mode de réalisation particulier, les charges pouvant être utilisées dans la présente invention une capacité d'absorption d'huile de 0,25 mL/g à 3,5 mL/g, de préférence de 0,93 mL/g à 2,5mL/g, voire de 1,25 mL/g à 2,5 mL/g.

Les tailles des charges peuvent être mesurées par diffusion statique de la lumière au moyen d'un granulomètre commercial de type MasterSizer 2000 de chez Malvern. Les données sont traitées sur la base de la théorie de diffusion de Mie. Cette théorie, exacte pour des particules isotropes, permet de déterminer dans le cas de particules non sphériques, un diamètre « effectif » de particules. Cette théorie est notamment décrite dans l'ouvrage de Van de Hulst, H.C., "Light Scattering by Small Particles," Chapitres 9 et 10, Wiley, New York, 1957.

Selon un mode de réalisation particulier, les charges pouvant être utilisées dans la présente invention présentent une taille exprimée en diamètre moyen en volume D(4,3) allant de 0,1 µm à 40 µm, de préférence de 0,5 µm à 20 µm, et encore plus préférentiellement de 1 µm à 16 µm.

Selon un mode de réalisation particulier, les charges utilisées dans la présente invention présentent une densité non tassée allant de 0,2 g/cm³ à 2,2 g/cm³.

Selon un mode de réalisation particulier, elles ont une surface spécifique allant de 30 à 1000 m²/g, et plus particulièrement de 150 à 800 m²/g.

On entend dans la présente demande par « particules sphériques » des particules ayant la forme ou sensiblement la forme d'une sphère, insolubles dans le milieu de la composition selon l'invention, même à la température de fusion du milieu (environ 100 °C).

Selon un mode de réalisation particulier de l'invention, les particules sphériques de silice poreuse sont des microparticules. De préférence, elles ont une taille exprimée en diamètre moyen en volume D(4,3) allant de 0,5 à 30 µm, plus particulièrement de 1 à 20 µm, et préférentiellement de 1 à 16 µm.

A titre d'exemple de microbilles de silice poreuse, on peut utiliser les produits commerciaux suivants : Silica Beads SB-150, SB-300 ou encore SB 700, préférentiellement SB 300 de la société MYOSHI KASEI ; la gamme des SUNSPHERE de la société Asahi Glass AGC SI-TECH notamment la Sunsphere H-51 ou encore les Sunsphère 12L, Sunsphère H-201, H-52 et H-53 ; Sunsil 130 de la société Sunjin ; Spherica P-1500 de la société Ikeda Corporation ; Sylosphere de la société Fuji Silysia ; les gammes des Silica Pearl et Satinier de la société JGC Catalysts and Chemicals, plus particulièrement la Satinier M13 et M16, les silices MSS-500 de la société KOBO, et plus particulièrement la MSS-500-20N, ainsi que la Silica Shells de la société KOBO.

Selon un mode de réalisation particulier, les particules sphériques de cellulose utilisables dans le cadre de l'invention sont des microparticules. De préférence, elles ont une taille exprimée en diamètre moyen en volume D(4,3) allant de 0,1 à 35 µm, de préférence de 1 à 20 µm, et plus particulièrement de 4 à 15 µm.

A titre d'exemples de microparticules sphériques de cellulose, on peut notamment citer les billes solides de cellulose commercialisées sous les dénominations CELLULOBEADS D-10, CELLULOBEADS D-5 et CELLULOBEADS USF par la société DAITO KASEI KOGYO.

Les acides aminés N-acylés comprennent un groupe acyle ayant de 8 à 22 atomes de carbones, comme par exemple un groupe 2-éthyl hexanoyle, caproyle, lauroyle, myristoyle, palmitoyle, stéaroyle, cocoyle. L'acide aminé peut être par exemple la lysine, l'acide glutamique, l'alanine, de préférence la lysine.

Selon un mode de réalisation particulier, le ou les acides aminés N-acylés comprennent un groupe acyle ayant de 10 à 14 atomes de carbone. De préférence, il s'agit du groupe lauroyle. Avantageusement, la poudre d'acide aminé N-acylé peut être une poudre de lauroyl lysine telle que celle qui est commercialisée sous la dénomination AMIHOPE LL par la société AJINOMOTO ou encore celle qui est commercialisée sous la dénomination CORUM 5105 S par la société CORUM.

Selon un mode de réalisation particulier de l'invention, les charges choisies parmi les particules sphériques de silice poreuse, les particules sphériques de cellulose, et les poudres d'acide aminé N-acylé ayant un groupe acyle en C8-C22 ont une granulométrie hétérogène, c'est-à-dire une grande répartition de taille pour une taille exprimée en diamètre moyen en volume donnée.

La composition conforme à l'invention comprend de 20 % à 50 % en poids, de préférence de 25 % à 35 % en poids du poids total de la composition, d'au moins une charge choisie parmi les particules sphériques de silice poreuse, les particules sphériques de cellulose, et les poudres d'acide aminé N-acylé ayant un groupe acyle en C8-C22.

Selon un mode de réalistion conforme à l'invention, la composition comprend de 20 % à 50 % en poids, de préférence de 25 % à 35 % en poids du poids total de la composition, d'une seule charge choisie parmi les particules sphériques de silice poreuse, les particules sphériques de cellulose, et les poudres d'acide aminé N-acylé ayant un groupe acyle en C8-C22.

Selon un premier mode de réalisation particulier, la composition conforme à l'invention comprend de 25 % à 50 % en poids, de préférence de 25 % à 35 % en poids du poids total de la composition, d'au moins une charge choisie parmi les particules sphériques de silice poreuse. Avantageusement, la composition conforme à l'invention peut comprendre de 25 % à 50 % en poids, de préférence de 25 % à 35 % en poids du poids total de la composition, d'une seule charge choisie parmi les particules sphériques de silice poreuse.

Selon un deuxième mode de réalisation particulier, la composition conforme à l'invention comprend de 25 % à 50 % en poids, de préférence de 25 % à 35 % en poids du poids total de la composition, d'au moins une charge choisie parmi les particules sphériques de cellulose. Avantageusement, la composition conforme à l'invention peut comprendre de 25 % à 50 % en poids, de préférence de 25 % à 35 % en poids du poids total de la composition, d'une seule charge choisie parmi les particules sphériques de cellulose.

Selon un troisième mode de réalisation particulier, la composition conforme à l'invention comprend de 25 % à 50 % en poids, de préférence de 25 % à 35 % en poids du poids total de la composition, d'au moins une charge choisie parmi les poudres d'acide aminé N-acylé ayant un groupe acyle en C8-C22. Avantageusement, la composition conforme à l'invention peut comprendre de 25 % à 50 % en poids, de préférence de 25 % à 35 % en poids du poids total de la composition, d'une seule charge choisie parmi les poudres d'acide aminé N-acylé ayant un groupe acyle en C8-C22

Selon un quatrième mode de réalisation, la composition conforme à l'invention comprend au moins deux charges choisies parmi les particules sphériques de silice poreuse, les particules sphériques de cellulose, et les poudres d'acide aminé N-acylé ayant un groupe acyle en C8-C22.

Selon un mode spécifique, la composition selon l'invention comprend au moins deux charges distinctes l'une de l'autre dont l'une est choisie parmi les particules de silice poreuse sphériques, en particulier les microparticules sphériques de silice poreuse, et l'autre est choisie parmi les particules sphériques de cellulose et les poudres d'acide aminé N-acylé ayant un groupe acyle en C8-C22.

En particulier, la composition peut comprendre au moins deux charges distinctes l'une de l'autre dont l'une est choisie parmi les particules sphériques de cellulose et l'autre est choisie parmi les particules sphériques de silice poreuse, en particulier les microparticules sphériques de silice poreuse.

La composition peut également comprendre au moins deux charges distinctes l'une de l'autre dont l'une est choisie parmi les poudres d'acide aminé N-acylé ayant un groupe acyle en C8-C22 et l'autre est choisie parmi les particules sphériques de silice poreuse, en particulier les microparticules sphériques de silice poreuse.

Selon un cinquième mode de réalisation particulier, la composition conforme à l'invention comprend au moins trois charges choisies parmi les particules sphériques de silice poreuse, les particules sphériques de cellulose, et les poudres d'acide aminé N-acylé ayant un groupe acyle en C8-C22.

Selon un mode spécifique, la composition conforme à l'invention comprend au moins trois charges distinctes les unes des autres dont dont la première est choisie parmi les particules sphériques de silice poreuse, en particulier les microparticules sphériques de silice poreuse, la deuxième est choisie parmi les particules sphériques de cellulose, et la troisième est choisie parmi les poudres d'acide aminé N-acylé ayant un groupe acyle en C8-C22.

Selon un mode particulier de réalisation la composition ne comprend pas de charge additionnelle différente des particules sphériques de silice poreuse, des particules sphériques de cellulose, et des poudres d'acide aminé N-acylé ayant un groupe acyle en C8-C22.

Alternativement, la composition conforme à l'invention peut en outre comprendre au moins une charge additionnelle différente des particules sphériques de silice poreuse, des particules sphériques de cellulose, et des poudres d'acide aminé N-acylé ayant un groupe acyle en C8-C22.

La ou les charges additionnelles peuvent être notamment des charges organiques et/ou des charges inorganiques.

La ou les charges additionnelles peuvent également être caractérisées par leur surface spécifique par unité de masse ou par unité de volume, leur taille exprimée en diamètre moyen en volume D(4,3), leur densité non tassée, et/ou leur capacité d'absorption d'huile. La définition et les méthodes de mesure de ces paramètres ont été décrites précédemment pour les charges choisies parmi les particules sphériques de silice poreuse, les particules sphériques de cellulose, et les poudres d'acide aminé N-acylé ayant un groupe acyle en C8-C22.

Selon un mode de réalisation particulier, la ou les charges additionnelles pouvant être utilisées dans la présente invention une capacité d'absorption d'huile de 0,25 mL/g à 3,5 g/g, de préférence de 0,93 mL/g à 2,5 mL/g, voire de 1,25 mL/g à 2,5 mL/g.

Selon un mode de réalisation particulier, la ou les charges additionnelles pouvant être utilisées dans la présente invention présentent une taille exprimée en diamètre moyen en volume D(4,3) allant de 0,1 µm à 40 µm, de préférence de 0,5 µm à 20 µm, et encore plus préférentiellement de 1 µm à 16 µm.

Selon un mode de réalisation particulier, la ou les charges additionnelles pouvant être utilisées dans la présente invention présentent une densité non tassée allant de 0,2 g/cm³ à 2,2 g/cm³.

### Charges organiques

On entend dans la présente demande par « charge organique », tout solide organique insoluble dans le milieu à température ambiante (25 °C).

On entend par « organique », tout composé ou polymère dont la structure chimique comprend au moins un ou plusieurs atomes de carbone.

Comme charges organiques susceptibles d'être utilisées dans la composition de l'invention, on peut citer par exemple, les particules de polyamide (nylon®) et notamment celles vendues sous les dénominations ORGASOL® par la société Atochem ; les poudres de polyéthylène ; les microsphères à base de copolymères acryliques, telles que celles en copolymère diméthacrylate d'éthylène glycol/ methacrylate de lauryle vendues par la société Dow Corning sous la dénomination de POLYTRAP® ; les microsphères de polyméthacrylate de méthyle, commercialisées sous la dénomination MICROSPHERE M-100® par la société Matsumoto ou sous la dénomination COVABEAD LH85® par la société Wackherr ; les poudres de copolymère éthylène-acrylate, comme celles commercialisées sous la dénomination FLOBEADS® par la société Sumitomo Seika Chemicals ; les poudres expansées telles que les microsphères creuses et notamment, les microsphères formées d'un terpolymère de chlorure de vinylidène, d'acrylonitrile et de méthacrylate et commercialisées sous la dénomination EXPANCEL® par la société Kemanord Plast sous les références 551 DE 12® (granulométrie d'environ 12 µm), 551 DE 20® (granulométrie d'environ 30 µm), 551 DE 50® (granulométrie d'environ 40 µm), ou les microsphères commercialisées sous la dénomination MICROPEARL F 80 ED® par la société Matsumoto ; les poudres de matériaux organiques naturels tels que les poudres de polysaccharide, et en particulier les poudres d'amidon, notamment d'amidons de maïs, de blé ou de riz, réticulés ou non, les poudres d'amidon réticulé par l'anhydride octénylsuccinate, commercialisées sous la dénomination DRY-FLO® par la société National Starch, les poudres d'amidon de maïs cireux telles que celles qui sont commercialisées sous les dénominations C* GEL 04201 par la société CARGILL, AMIDON DE MAIS B par la société ROQUETTE, et ORGANIC CORN STARCH par la société DRACO NATURAL PRODUCTS ; les microbilles de résine de silicone telles que celles commercialisées sous la dénomination TOSPEARL par la société Toshiba Silicone, notamment TOSPEARL 240 ; les particules de microdispersion de cire, qui ont de préférence des dimensions moyennes inférieures à 25 µm, et notamment allant de 0,5 µm à 25 µm, et qui sont constituées essentiellement d'une cire ou d'un mélange de cires, telles que les produits commercialisés sous la dénomination Aquacer par la société Byk Cera, et notamment : Aquacer 520 (mélange de cires synthétiques et naturelles), Aquacer 514® ou 513® (cire de polyéthylène), Aquacer 511® (cire polymérique), ou telles que les produits commercialisés sous la dénomination Jonwax 120 par la société Johnson Polymer (mélange de cires de polyéthylène et de paraffine) et sous la dénomination Ceraflour 961 ® par la société Byk Cera (cire de polyéthylène modifiée micronisée) ; et leurs mélanges.

### Charges inorganiques

On entend dans la présente demande par « charge inorganique », tout solide inorganique insoluble dans le milieu à température ambiante (25 °C).

On entend par « inorganique », tout composé ou polymère dont la structure chimique ne comprend pas d'atome de carbone.

Comme exemples de charges inorganiques additionnelles, on peut citer les zéolites tels que les produits commercialisés par la société ZEOCHEM sous les dénominations ZEOFLAIR 300, ZEOFLAIR 200, ZEOFLAIR 100, X-MOL et X-MOL MT.

Une zéolithe, ou zéolite est un cristal formé d'un squelette microporeux d'alumino-silicate, dont les espaces vides connexes sont initialement occupés par des cations et des molécules d'eau. On les qualifie aussi de tamis moléculaires.

On peut encore citer le calcium magnésium carbonate tel que ceux qui sont commercialisés par la société IMERYS sous la dénomination CALCIDOL, par la société LCW (Sensient) sous la dénomination CARBOMAT, par la société OMYA sous la dénomination OMYACARE S 60-AV.

On peut également citer les particules inorganiques lamellaires comme les talcs, les micas, les nacres et leurs mélanges.

Les talcs sont des silicates de magnésium hydratés comprenant le plus souvent du silicate d'aluminium. La structure cristalline du talc consiste en des couches répétées d'un sandwich de brucite entre des couches de silice.

Plus particulièrement, les particules lamellaires sont choisies parmi les talcs.

De manière avantageuse, on utilise plus particulièrement dans la composition de l'invention, comme particules lamellaires le talc tel que ceux commercialisés sous les dénominations LUZENAC PHARMA M et UM par la société IMERYS, ROSE TALC et TALC SG-2000 par la société Nippon Talc ; le mica tel que ceux commercialisés sous les dénominations MICA M RP et SILK MICA par la société Merck ; les mica-titanes tels que le mica-oxyde de titane-oxyde de fer brun (CTFA : Mica / Iron oxides / Titanium dioxide) commercialisé sous la dénomination CLOISONNE ROUGE FLAMBE 440 X par la société Engelhard. A titre de mica, on peut citer le Mica commercialisé sous la dénomination Sericite S-152-BC par la société MYOSHI KASEI.

On peut citer parmi les charges inorganiques, les particules de perlite, et de préférence les particules de perlite expansées.

Les perlites utilisables selon l'invention sont généralement des aluminosilicates d'origine volcanique et ont comme composition :
70,0-75,0% en poids de silice SiO₂ ;
12,0-15,0% en poids d'oxyde d'aluminium oxyde Al₂O₃ ;
3,0-5,0% d'oxyde de sodium Na₂O ;
3,0-5,0% d'oxyde de potassium K₂O ;
0,5-2% d'oxyde de fer Fe₂O₃ ;
0,2-0,7% d'oxyde de magnésium MgO ;
0,5-1,5% d'oxyde de calcium CaO ;
0,05 - 0,15% d'oxyde de titane TiO₂.

On peut citer notamment les perlites commercialisées sous les dénominations OPTIMAT 2550 OR par la société WOELD MINERALS, EUROPERL EMP-2 et EUROPERL 1 par la société IMERYS.

Lorsque la composition conforme à l'invention comprend au moins une charge additionnelle différente des charges choisies parmi les particules sphériques de silice poreuse, les particules sphériques de cellulose, et les poudres d'acide aminé N-acylé ayant un groupe acyle en C8-C22, elle(s) est (sont) de préférence présente(s) en quantité au moins égale à 0,1 % en poids, et encore plus préférentiellement entre 5 % et 25 % en poids du poids total de la composition. Selon un mode particulier de réalisation, la teneur en charges additionnelles lorsque elles sont présentes va de 0,1 à 10% en poids, de préférence de 0,1 à 5% en poids, par rapport au poids total de la composition.

Selon un mode de réalisation particulier, lorsque la composition comprend au moins deux charges distinctes dont l'une est choisie parmi les particules sphériques de silice poreuse, le rapport massique R (particules sphériques de silice poreuse) / (autres charges) est supérieur ou égal à 0,75, de préférence compris entre 0,75 et 3.

### Phase grasse

La composition conforme à l'invention comprend de 40 % à 85 % en poids du poids total de la composition d'une phase grasse.

Selon un mode de réalisation particulier, la composition conforme à l'invention comprend de 45 % à 85 % en poids d'une phase grasse. De préférence, la proportion de la phase grasse va de 45 % à 75 % en poids, et encore plus préférentiellement de 57 % à 70 % en poids du poids total de la composition.

Cette quantité indiquée ne comprend pas la teneur en gélifiants lipophiles tels que ceux qui sont décrits ci-dessus.

Au sens de l'invention, la phase grasse inclut tout corps gras liquide à température ambiante et pression atmosphérique, généralement des huiles, ou solide à température ambiante et pression atmosphérique, à l'image des cires, ou tout composé pâteux, présents dans ladite composition.

Le ou les corps gras présents dans la composition peuvent être choisis par l'homme du métier sur base de ses connaissances générales, de manière à conférer à la composition finale les propriétés souhaitées, notamment en termes de propriétés rhéologiques (mesure de pénétrométrie, écoulement, consistance), en termes de texture et de stabilité.

La phase grasse de la composition conforme à l'invention comprend au moins 20 % en poids d'au moins une huile choisie parmi les huiles hydrocarbonées d'origine végétale, de préférence les triglycérides, les alcools de Guerbet et les esters d'acide gras en C8-C30 et d'alcool de Guerbet, les éthers d'alcools gras comportant de 6 à 20 atomes de carbone.

Selon un mode de réalisation particulier, la phase grasse de la composition conforme à l'invention comprend au moins 25 % en poids d'au moins une huile choisie parmi les huiles hydrocarbonées d'origine végétale, de préférence les triglycérides, les alcools de Guerbet et les esters d'acide gras en C8-C30 et d'alcool de Guerbet.

Selon un autre mode de réalisation particulier, la phase grasse de la composition conforme à l'invention comprend au moins 25 % en poids d'au moins deux huiles choisies parmi les huiles hydrocarbonées d'origine végétale, de préférence les triglycérides, les alcools de Guerbet et les esters d'acide gras en C8-C30 et d'alcool de Guerbet.

Par huiles, on entend les corps gras liquides à température ambiante (25 °C) et pression atmosphérique.

A titre d'huiles hydrocarbonées d'origine végétale, on peut citer le squalane, les triglycérides liquides d'acides gras comportant de 4 à 30 atomes de carbone comme les triglycérides des acides heptanoïque ou octanoïque ou encore, par exemple, les huiles de jojoba, de babassu, de tournesol, d'olive, de noix de coco, de noix du brésil, de marula, de maïs, de soja, de courge, de pépins de raisin, de lin, de sésame, de noisette, d'abricot, de macadamia, d'arara, de coriandre, de ricin, d'avocat, les triglycérides des acides caprylique/caprique comme ceux commercialisés par la société Stearineries Dubois ou ceux commercialisés sous les dénominations Miglyol 810, 812 et 818 par la société Dynamit Nobel, l'huile de beurre de karité.

A titre d'alcools de Guerbet ou d'esters d'acides gras en C8-C30 et d'alcools de Guerbet, on peut notamment citer l'octyldodécanol et les esters d'octyldodécanol et d'acide gras en C8-C30, tels que le myristate d'octyldodécyle. On peut citer en particulier l'octydodécanol tel que celui qui est commercialisé sous la dénomination EUTANOL G par la société COGNIS (BASF) et le myristate d'octyldodécyle commercialisé sous la dénomination DUB MOD par la société GATTEFOSSE.

Les éthers d'alcools gras pouvant être utilisés dans le cadre de l'invention sont des éthers d'alcools gras comprenant de 6 à 20 atomes de carbone, de préférence de 8 à 12, et encore plus préférentiellement de 8 à 10.

Ces éthers peuvent être obtenus à partir de deux alcools gras différents ou de deux alcools gras identiques. De préférence, ils sont obtenus à partir de deux alcools gras identiques tel que l'alcool caprylique (encore appelé octan-1-ol ou n-octanol). L'éther correspondant est alors le dicaprylyl éther tel que celui qui est commercialisé sous la dénomination Cetiol OE par la société Cognis.

### Huiles additionnelles

Selon un mode de réalisation particulier, la composition selon l'invention comprend au moins une huile additionnelle différente des huiles choisies parmi les huiles hydrocarbonées d'origine végétale, lesalcools de Guerbet, les esters d'acide gras en C8-C30 et d'alcool de Guerbet.

Comme huiles utilisables dans la composition de l'invention, on peut citer par exemple :
- les esters et les éthers de synthèse, notamment d'acides et/ ou d'alcools gras, comme les huiles de formules R¹COOR² et R¹OR² dans laquelle R¹CO représente le reste d'un acide gras ou R¹ représente le reste d'un alcool gras comportant de 8 à 29 atomes de carbone, et R² représente une chaîne hydrocarbonée, ramifiée ou non, contenant de 3 à 30 atomes de carbone, comme par exemple l'huile de Purcellin, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrytyle ;
- leurs mélanges.

On peut citer également les huiles suivantes :
- les esters issus de la réaction d'au moins un acide gras comportant au moins 6 atomes de carbone, de préférence de 6 à 26 atomes de carbone, et mieux de 6 à 20 atomes de carbone, encore mieux de 6 à 16 atomes de carbone et d'au moins un alcool comprenant de 1 à 17 atomes de carbone et mieux de 3 à 15 atomes de carbone ; on peut citer notamment le myristate d'isopropyle tel que ceux qui sont commercialisés sous la dénomination PALMESTER 1510 par la société KLK OLEO, sous la dénomination LEXOL IPM-NF par la société INOLEX CHEMICAL COMPANY ou encore sous la dénomination ISOPROPYLMYRISTATE par la société COGNIS (BASF), l'isostéarate d'isopropyle tel que celui qui est commercialisé sous la dénomination RADIA 7739 par la société OLEON, le palmitate d'isopropyle, le caprate/caprylate d'ethyl-.2-hexyle (ou caprate/caprylate d'octyle), le palmitate d'éthyl-2-hexyle, le néopentanoate d'isostéaryle, l'isononanoate d'isononyle, le laurate d'hexyle, les esters d'acide lactique et d'alcools gras comprenant 12 ou 13 atomes de carbone, le carbonate de dicaprylyle tel que celui qui est commercialisé sous la dénomination CETIOL CC par la société COGNIS,
- les éthers de glycérol comprenant de 6 à 12 atomes de carbone comme le 2-éthyl hexyle éther de glycérol (nom INCI : ethylhexylglycerin) tel que le Sensiva SC 50 de la société Schulke & Mayr GmbH.

On peut en particulier citer le mélange d'esters d'acides caprylique/caprique et d'alcools gras en C12-C18 tel que le coco-caprylate/ caprate commercialisé sous la dénomination CETIOL LC par la société COGNIS ou encore sous la dénomination DUB 810 C par la société STEARINERIE DUBOIS ;
- les polyesters obtenus par condensation de dimère et/ou trimère d'acide gras insaturé en C8-C30 et de diol comme par exemple les polyesters d'acide dilinoléique et de diol commercialisés par Biosynthis sous la dénomination Viscoplast et notamment le polymère portant le nom INCI dilinoleic acid/propanediol copolymer ;
- et leurs mélanges.

La composition selon l'invention peut présenter une teneur totale en huiles allant de 40 % à 85 % en poids, de préférence de 45 % à 75 % en poids, mieux de 57 % à 70 % par rapport au poids total de la composition.

La composition conforme à l'invention peut également comprendre au moins un corps gras solide tel que les composés pâteux et les cires.

### Composé pâteux

Par "composé pâteux" au sens de la présente invention, on entend un composé gras lipophile à changement d'état solide/liquide réversible et comportant à la température de 23 °C une fraction liquide et une fraction solide.

Un composé pâteux est à la température de 23 °C, sous la forme d'une fraction liquide et d'une fraction solide. En d'autres termes, la température de fusion commençante du composé pâteux est inférieure à 23 °C. La fraction liquide du composé pâteux, mesurée à 23 °C, représente de 20 à 97 % en poids du composé pâteux. Cette fraction liquide à 23 °C représente plus préférentiellement de 25 à 85 %, et mieux de 30 à 60 % en poids du composé pâteux.

La fraction liquide en poids du composé pâteux à 23 °C est égale au rapport de l'enthalpie de fusion consommée à 23 °C sur l'enthalpie de fusion du composé pâteux.

L'enthalpie de fusion consommée à 23 °C est la quantité d'énergie absorbée par l'échantillon pour passer de l'état solide à l'état qu'il présente à 23 °C constitué d'une fraction liquide et d'une fraction solide.

L'enthalpie de fusion du composé pâteux est l'enthalpie consommée par le composé pour passer de l'état solide à l'état liquide. Le composé pâteux est dit à l'état solide lorsque l'intégralité de sa masse est sous forme solide. Le composé pâteux est dit à l'état liquide lorsque l'intégralité de sa masse est sous forme liquide.

L'enthalpie de fusion du composé pâteux est égale à l'aire sous la courbe du thermogramme obtenu à l'aide d'un calorimètre à balayage différentiel (D. S. C), tel que le calorimètre vendu sous la dénomination MDSC 2920 par la société TA instrument, avec une montée en température de 5 ou 10 °C par minute, selon la norme ISO 11357-3:1999. L'enthalpie de fusion du composé pâteux est la quantité d'énergie nécessaire pour faire passer le composé de l'état solide à l'état liquide. Elle est exprimée en J/g.

La fraction liquide du composé pâteux, mesurée à 32 °C, représente de préférence de 40 à 100 % en poids du composé pâteux, mieux encore de 50 à 100 % en poids du composé pâteux. Lorsque la fraction liquide du composé pâteux mesurée à 32 °C est égale à 100 %, la température de la fin de la plage de fusion du composé pâteux est inférieure ou égale à 32 °C.

La fraction liquide du composé pâteux, mesurée à 32 °C, est égale au rapport de l'enthalpie de fusion consommée à 32 °C sur l'enthalpie de fusion du composé pâteux. L'enthalpie de fusion consommée à 32 °C est calculée de la même façon que l'enthalpie de fusion consommée à 23 °C.

Le composé pâteux a de préférence une dureté à 20 °C allant de 0,001 à 0,5 MPa, de préférence de 0,002 à 0,4 MPa.

La dureté est mesurée selon une méthode de pénétration d'une sonde dans un échantillon de composé et en particulier à l'aide d'un analyseur de texture (par exemple le TA-XT2i de chez Rhéo) équipé d'un cylindre en inox de 2 mm de diamètre. La mesure de dureté est effectuée à 20 °C au centre de 5 échantillons. Le cylindre est introduit dans chaque échantillon, la profondeur de pénétration étant de 0,3 mm. La valeur relevée de la dureté est celle du pic maximum.

Le composé pâteux est choisi parmi les composés d'origine végétale. Un composé pâteux peut être obtenu par synthèse à partir de produits de départ d'origine végétale.

Le composé pâteux peut être choisi notamment parmi l'huile de jojoba isomérisée telle que l'huile de jojoba partiellement hydrogénée isomérisée trans fabriquée ou commercialisée par la société Desert Whale sous la référence commerciale Iso-Jojoba-50®, la cire d'orange comme, par exemple, celle qui est commercalisée sous la référence Orange Peel Wax par la société Koster Keunen, le beurre de cupuacu (Rain forest RF3410 de la société Beraca Sabara), le beurre de murumuru (RAIN FOREST RF3710 de la société Beraca Sabara), le beurre de karité, l'huile d'olive partiellement hydrogénée comme, par exemple, le composé commercialisé sous la référence Beurrolive par la société Soliance, le beurre de cacao, l'huile de mangue comme, par exemple, la Lipex 203 de la société Aarhuskarlshamn et leurs mélanges.

On peut également citer les mélanges d'acides gras en C8-C30 et d'alcool gras en C8-C30 tels les mélanges d'alcool laurique et méthyl laurate, d'alcool stéarique et méthyl palmitate ou méthyl béhénate telle la gamme des Purester commercialisée par la société Strahl & Pitsch, notamment le lauryl laurate connu sous la dénomination commerciale de Purester 24.

De même, on pourra citer les esters d'acide gras en C8-C30 et de polyglycérol comprenant de 2 à 10 unités glycéryles, tels que le Polyglyceryl-3 polyricinoleate commercialisé par la société Aarhuskarlshamn sous la dénomination Akoline PGPR ou encore un mélange de 3 cires jojoba esters & helianthus annus seed wax & acacia decurrens extract et de polyglycéryle tel le produit commercialisé sous la dénomination d'Hydracire S ou Acticire® par la société Gatefossé.

On peut également citer les esters de glycérol hydrogénés tels que par exemple le produit commercialisé sous la dénomination Cegesoft HF 52 par la société COGNIS (BASF), mélange d'huiles de colza et de Palme hydrogénées ou encore le produit commercialisé sous la dénomination Softisan 100 Cremer par la société OLEO.

On peut également citer les mélanges de mono et/ou diester d'acide gras en C8-C18 et de mono ou polyglycérol tel que le stéarate de glycéryle comme par exemple le glyceryl stearate commercialisé sous la référence CUTINA GMS V par la société Cognis ou encore le mélange de mono et distearate de glycéryle commercialisé par la société STEARINERIE DUBOIS sous la dénomination DUB GMS 50/50,

Lorsqu'ils sont présents, la quantité de composés pâteux peut aller par exemple de 0,05 % à 85 % en poids, mieux de 0,1 à 40 % en poids, en particulier de 0,5 à 10 % en poids par rapport au poids total de la composition.

### Cires

Par cire au sens de la présente invention, on entend un composé lipophile, solide à température ambiante (25 °C), à changement d'état solide/liquide réversible, ayant une température de fusion supérieure ou égale à 30 °C, et pouvant aller jusqu'à 120 °C. En particulier, les cires présentent une température de fusion supérieure à 30 °C, et mieux supérieure à 45 °C.

Au sens de l'invention, la température de fusion correspond à la température du pic le plus endothermique observé en analyse thermique (analyse calorimétrique différentielle ou DSC) telle que décrite dans la norme ISO 11357-3 ; 1999. Le point de fusion de la cire peut être mesuré à l'aide d'un calorimètre à balayage différentiel (DSC), par exemple le calorimètre vendu sous la dénomination « MDSC 2920 » par la société TA Instruments.

Le protocole de mesure est le suivant :

Un échantillon de 5 mg de cire disposé dans un creuset est soumis à une première montée en température allant de - 20 °C à 100 °C, à la vitesse de chauffe de 10 °C/minute, puis est refroidi de 100 °C à - 20 °C à une vitesse de refroidissement de 10 °C/minute et enfin soumis à une deuxième montée en température allant de - 20 °C à 100 °C à une vitesse de chauffe de 5 °C/minute. Pendant la deuxième montée en température, on mesure la variation de la différence de puissance absorbée par le creuset vide et par le creuset contenant l'échantillon de cire en fonction de la température. Le point de fusion du composé est la valeur de la température correspondant au sommet du pic de la courbe représentant la variation de la différence de puissance absorbée en fonction de la température.

Par cire dure, on entend au sens de la présente invention, une cire présentant à 20 °C, une dureté supérieure à 5 MPa, notamment allant de 5 à 30 MPa, de préférence supérieure à 6 MPa, mieux encore allant de 6 à 25 MPa.

La dureté de la cire est déterminée par la mesure de la force en compression mesurée à 20 °C à l'aide du texturomètre vendu sous la dénomination TA-XT2 par la société RHEO, équipé d'un cylindre en inox d'un diamètre de 2 mm se déplaçant à la vitesse de mesure de 0,1 mm/s, et pénétrant dans la cire à une profondeur de pénétration de 0,3 mm.

Le protocole de mesure est le suivant : on fait fondre la cire à une température égale à la température de fusion de la cire + 10 °C. On fait couler la cire fondue dans un récipient de 25 mm de diamètre et de 20 mm de profondeur. La cire est recristallisée à température ambiante (25 °C) pendant 24 heures de telle sorte que la surface de la cire soit plane et lisse, puis la cire est conservée pendant au moins 1 heure à 20 °C avant d'effectuer la mesure de la dureté ou du collant.

Le mobile du texturomètre est déplacé à la vitesse de 0,1 mm/s, puis pénètre dans la cire jusqu'à une profondeur de pénétration de 0,3 mm. Lorsque le mobile a pénétré dans la cire à la profondeur de 0,3 mm, le mobile est maintenu fixe pendant 1 seconde (correspondant au temps de relaxation) puis est retiré à la vitesse de 0,5 mm/s.

La valeur de la dureté est la force de compression maximale mesurée divisée par la surface du cylindre du texturomètre en contact avec la cire.

Comme cire, on peut utiliser avantageusement les cires d'origine végétale telles que la cire d'abeille, notamment celle qui est commercialisée sous la dénomination WHITE BEESWAX SP 453P par la société STRAHL & PITSCH ou encore CERABEIL LOR par la société BAERLOCHER, la cire de blé noir telle que celle qui est commercialisée par la société CODIF, la cire de carnauba, la cire de candellila, notamment la référence commerciale CANDELILLA WAX SP 75 G de la société STRAHL & PITSCH, la cire de jojoba hydrogénée, la cire de sumac, les cires obtenues par hydrogénation d'huile d'olive estérifiée avec les alcool gras à chaîne en C12 à C18 vendues par la société SOPHIM dans la gamme Phytowax (12L44, 14L48, 16L55 et 18L57), la cire de son de riz, les alcools cétylique, stéarylique et béhénique, la cire de laurier, la cire d'Ouricury.

On peut également utiliser au moins un ester d'acide béhénique et de glycérol, et en particulier un mélange d'esters d'acide béhénique et de glycérol comme par exemple le mélange glyceryl dibehenate, tribehenin, glyceryl behenate commercialisé par la société Gattefossé sous la référence COMPRITOL 888 CG ATO.

Lorsqu'elles sont présentes, les cires peuvent être présentes en une teneur allant de 0,01 à 40 % en poids, de préférence de 0,05 à 10 % en poids, et mieux de 0,1 à 5 % en poids par rapport au poids total de la composition.

La phase grasse de la composition conforme à l'invention comprend au moins une huile choisie parmi les huiles hydrocarbonées d'origine végétale, de préférence les triglycérides, les alcools de Guerbet, et esters d'acide gras en C8-C30 et d'alcool de Guerbet, les éthers d'alcools gras comportant de 6 à 20 atomes de carbone, de préférence choisie parmi les huiles hydrocarbonées d'origine végétale, de préférence les triglycérides, es alcools de Guerbet, les esters d'acide gras en C8-C30 et d'alcool de Guerbet.

Selon un mode de réalisation particulier de l'invention, la composition comprend de 40 % à 85 % en poids d'au moins une huile choisie parmi les huiles hydrocarbonées d'origine végétale, de préférence les triglycérides, les alcools de Guerbet, les esters d'acide gras en C8-C30 et d'alcool de Guerbet, les éthers d'alcools gras comportant de 6 à 20 atomes de carbone, de préférence choisie parmi les huiles hydrocarbonées d'origine végétale, de préférence les triglycérides, les alcools de Guerbet, les esters d'acide gras en C8-C30 et d'alcool de Guerbet.

Selon un premier mode de réalisation spécifique, la composition comprend de 40 % à 85 % en poids, de préférence de 45 % à 75 % en poids par rapport au poids total de la composition, d'au moins une huile choisie parmi les huiles hydrocarbonées d'origine végétale, de préférence les triglycérides, les alcools de Guerbet et les esters d'acide gras en C8-C30 et d'alcool de Guerbet.

Selon un deuxième mode de réalisation spécifique, la composition comprend de 40 % à 85 % en poids, de préférence de 45 % à 75 % en poids par rapport au poids total de la composition, d'au moins une huile choisie parmi les huiles hydrocarbonées d'origine végétale, de préférence les triglycérides, les alcools de Guerbet et les esters d'acide gras en C8-C30 et d'alcool de Guerbet, et de 5 % à 25 % en poids, de préférence de 5 % à 10 % en poids par rapport au poids total de la composition.

Selon un premier mode de réalisation spécifique de l'invention, la composition anhydre comprend :
- de 3 % à 15 % en poids d'au moins un gélifiant lipophile choisi parmi les esters de dextrine et d'acide gras, de préférence le palmitate de dextrine ;
- de 20 % à 50 % en poids, de préférence de 30 % à 40 % en poids d'au moins une charge, de préférence une seule charge, choisie parmi les particules sphériques de silice poreuse ; et
- de 40 % à 85 % en poids d'au moins une phase grasse contenant au moins 25 % en poids d'au moins une huile choisie parmi les huiles hydrocarbonées d'origine végétale, de préférence les triglycérides, les alcools de Guerbet, les esters d'acide gras en C8-C30 et d'alcool de Guerbet, les éthers d'alcools gras comportant de 6 à 20 atomes de carbone, les quantités en poids étant données par rapport au poids total de la composition.

Selon un mode de réalisation particulier, la composition anhydre comprend :
- de 3 % à 15 % en poids d'au moins un gélifiant lipophile choisi parmi les esters de dextrine et d'acide gras, de préférence le palmitate de dextrine ;
- de 20 % à 50 % en poids, de préférence de 30 % à 40 % en poids d'au moins une charge, de préférence une seule charge, choisie parmi les particules sphériques de silice poreuse ; et
- de 40 % à 85 % en poids d'au moins une phase grasse contenant au moins 25 % en poids d'au moins une huile choisie parmi les alcools de Guerbet et les esters d'acide gras en C8-C30 et d'alcool de Guerbet ;
les quantités en poids étant données par rapport au poids total de la composition.

De préférence, la composition anhydre comprend :
- de 3 % à 15 % en poids d'au moins un gélifiant lipophile choisi parmi les esters de dextrine et d'acide gras, de préférence le palmitate de dextrine ;
- de 30 % à 40 % en poids d'au moins une charge, de préférence une seule charge, choisie parmi les particules sphériques de silice poreuse ; et
- de 40 % à 85 % en poids d'au moins une phase grasse contenant au moins 30 % en poids d'au moins une huile choisie parmi les alcools de Guerbet et les esters d'acide gras en C8-C30 et d'alcool de Guerbet ; les quantités en poids étant données par rapport au poids total de la composition.

Selon un mode de réalisation particulier, la composition anhydre comprend :
- de 3 % à 15 % en poids d'au moins un gélifiant lipophile choisi parmi les esters de dextrine et d'acide gras, de préférence le palmitate de dextrine ;
- de 20 % à 50 % en poids, de préférence de 30 % à 40 % en poids d'au moins une charge, de préférence une seule charge, choisie parmi les particules sphériques de silice poreuse ; et
- de 40 % à 85 % en poids d'au moins une phase grasse contenant au moins 25 % en poids d'au moins une huile choisie parmi les huiles hydrocarbonées d'origine végétale, de préférence les triglycérides ; les quantités en poids étant données par rapport au poids total de la composition.

De préférence, la composition anhydre comprend :
- de 3 % à 15 % en poids d'au moins un gélifiant lipophile choisi parmi les esters de dextrine et d'acide gras, de préférence le palmitate de dextrine ;
- de 30 % à 40 % en poids d'au moins une charge, de préférence une seule charge, choisie parmi les particules sphériques de silice poreuse ; et
- de 40 % à 85 % en poids d'au moins une phase grasse contenant au moins 30 % en poids d'au moins une huile choisie parmi les huiles hydrocarbonées d'origine végétale, de préférence les triglycérides ;
les quantités en poids étant données par rapport au poids total de la composition.

Selon un mode de réalisation particulier, la composition anhydre comprend :
- de 3 % à 15 % en poids d'au moins un gélifiant lipophile choisi parmi les esters de dextrine et d'acide gras, de préférence le palmitate de dextrine ;
- de 20 % à 50 % en poids, de préférence de 30 % à 40 % en poids d'au moins une charge choisie parmi les particules sphériques de silice poreuse ; et
- de 40 % à 85 % en poids d'au moins une phase grasse contenant au moins 25 % en poids d'au moins deux huiles dont l'une est choisie parmi les huiles hydrocarbonées d'origine végétale, de préférence les triglycérides ; les quantités en poids étant données par rapport au poids total de la composition.

De préférence, la composition anhydre comprend :
- de 3 % à 15 % en poids d'au moins un gélifiant lipophile choisi parmi les esters de dextrine et d'acide gras, de préférence le palmitate de dextrine ;
- de 30 % à 40 % en poids d'au moins une charge choisie parmi les particules sphériques de silice poreuse ; et
- de 40 % à 85 % en poids d'au moins une phase grasse contenant au moins 25 % en poids d'au moins une huile choisie parmi les huiles hydrocarbonées d'origine végétale, de préférence les triglycérides ;
les quantités en poids étant données par rapport au poids total de la composition.

Selon un deuxième mode de réalisation spécifique de l'invention, la composition anhydre comprend :
- de 3 % à 15 % en poids d'au moins un gélifiant lipophile choisi parmi les tri-esters d'acide gras en C8-C30 et de mono ou polyglycérol tels que le tri-hydroxy-stéarate de glycéryle ;
- de 20 % à 50 % en poids, de préférence de 30 % à 40 % en poids d'au moins une charge choisie parmi les poudres d'acide aminé N-acylé ayant un groupe acyle en C8-C22 ; et
- de 40 % à 85 % en poids d'au moins une phase grasse contenant au moins 25 % en poids d'au moins une huile choisie parmi les huiles hydrocarbonées d'origine végétale, de préférence les triglycérides, les alcools de Guerbet, les esters d'acide gras en C8-C30 et d'alcool de Guerbet, les éthers d'alcools gras comportant de 6 à 20 atomes de carbone ;
les quantités en poids étant données par rapport au poids total de la composition.

Selon un mode de réalisation particulier, la composition anhydre comprend :
- de 3 % à 15 % en poids d'au moins un gélifiant lipophile choisi parmi les tri-esters d'acide gras en C8-C30 et de mono ou polyglycérol tels que le tri-hydroxy-stéarate de glycéryle ;
- de 20 % à 50 % en poids, de préférence de 30 % à 40 % en poids d'au moins une charge choisie parmi les poudres d'acide aminé N-acylé ayant un groupe acyle en C8-C22 ; et
- de 40 % à 85 % en poids d'au moins une phase grasse contenant au moins 25 % en poids d'au moins deux huiles dont l'une est choisie parmi les huiles hydrocarbonées d'origine végétale, de préférence les triglycérides ;
les quantités en poids étant données par rapport au poids total de la composition.

De préférence, la composition anhydre comprend :
- de 3 % à 15 % en poids d'au moins un gélifiant lipophile choisi parmi les tri-esters d'acide gras en C8-C30 et de mono ou polyglycérol tels que le tri-hydroxy-stéarate de glycéryle ;
- de 30 % à 40 % en poids d'au moins une charge choisie parmi les poudres d'acide aminé N-acylé ayant un groupe acyle en C8-C22 ; et
- de 45 % à 85 % en poids d'au moins une phase grasse contenant au moins 40 % en poids d'au moins une huile choisie parmi les huiles hydrocarbonées d'origine végétale, de préférence les triglycérides ;
les quantités en poids étant données par rapport au poids total de la composition.

Selon un mode de réalisation particulier, la composition anhydre comprend :
- de 3 % à 15 % en poids d'au moins un gélifiant lipophile choisi parmi les tri-esters d'acide gras et de mono ou polyglycérol tels que le tri-hydroxy-stéarate de glycéryle ;
- de 20 % à 50 % en poids, de préférence de 30 % à 40 % en poids d'au moins une charge choisie parmi les poudres d'acide aminé N-acylé ayant un groupe acyle en C8-C22 ; et
- de 40 % à 85 % en poids d'au moins une phase grasse contenant au moins 25 % en poids d'au moins une huile choisie parmi les éthers d'alcools gras comportant de 6 à 20 atomes de carbone ;
les quantités en poids étant données par rapport au poids total de la composition.

De préférence, la composition anhydre comprend :
- de 3 % à 15 % en poids d'au moins un gélifiant lipophile choisi parmi les tri-esters d'acide gras et de mono ou polyglycérol tels que le tri-hydroxy-stéarate de glycéryle ;
- de 30 % à 40 % en poids d'au moins une charge choisie parmi les poudres d'acide aminé N-acylé ayant un groupe acyle en C8-C22 ; et
- de 40 % à 85 % en poids d'au moins une phase grasse contenant au moins 25 % en poids d'au moins une huile choisie parmi les éthers d'alcools gras comportant de 6 à 20 atomes de carbone ;
les quantités en poids étant données par rapport au poids total de la composition.

Selon un troisième mode de réalisation spécifique de l'invention, la composition anhydre comprend :
- de 3 % à 15 % en poids d'au moins un gélifiant lipophile choisi parmi les esters de dextrine et d'acide gras, de préférence le myristate de dextrine ;
- de 20 % à 50 % en poids, de préférence de 30 % à 40 % en poids d'au moins une charge choisie parmi les poudres d'acide aminé N-acylé ayant un groupe acyle en C8-C22 ; et
- de 40 % à 85 % en poids d'au moins une phase grasse contenant au moins 25 % en poids d'au moins une huile choisie parmi les huiles hydrocarbonées d'origine végétale, de préférence les triglycérides, les alcools de Guerbet, les esters d'acide gras en C8-C30 et d'alcool de Guerbet, les éthers d'alcools gras comportant de 6 à 20 atomes de carbone ;
les quantités en poids étant données par rapport au poids total de la composition.

Selon un mode de réalisation particulier, la composition anhydre comprend :
- de 3 % à 15 % en poids d'au moins un gélifiant lipophile choisi parmi les esters de dextrine et d'acide gras, de préférence le myristate de dextrine ;
- de 20 % à 50 % en poids, de préférence de 30 % à 40 % en poids d'au moins une charge choisie parmi les poudres d'acide aminé N-acylé ayant un groupe acyle en C8-C22 ; et
- de 40 % à 85 % en poids d'au moins une phase grasse contenant au moins 25 % en poids d'au moins une huile choisie parmi les alcools de Guerbet et les esters d'acide gras en C8-C30 et d'alcool de Guerbet ;
les quantités en poids étant données par rapport au poids total de la composition.

De préférence, la composition anhydre comprend :
- de 3 % à 15 % en poids d'au moins un gélifiant lipophile choisi parmi les esters de dextrine et d'acide gras, de préférence le myristate de dextrine ;
- de 30 % à 40 % en poids d'au moins une charge choisie parmi les poudres d'acide aminé N-acylé ayant un groupe acyle en C8-C22 ; et
- de 50 % à 85 % en poids d'au moins une phase grasse contenant au moins 50 % en poids d'au moins une huile choisie parmi les alcools de Guerbet et les esters d'acide gras en C8-C30 et d'alcool de Guerbet ;
les quantités en poids étant données par rapport au poids total de la composition.

Selon un mode de réalisation particulier, la composition anhydre comprend :
- de 3 % à 15 % en poids d'au moins un gélifiant lipophile choisi parmi les esters de dextrine et d'acide gras, de préférence le myristate de dextrine ;
- de 20 % à 50 % en poids, de préférence de 20 % à 30 % en poids d'au moins une charge choisie parmi les poudres d'acide aminé N-acylé ayant un groupe acyle en C8-C22 ; et
- de 40 % à 85 % en poids d'au moins une phase grasse contenant au moins 25 % en poids d'au moins une huile choisie parmi les huiles hydrocarbonées d'origine végétale, de préférence les triglycérides ;
les quantités en poids étant données par rapport au poids total de la composition.

De préférence, la composition anhydre comprend :
- de 3 % à 15 % en poids d'au moins un gélifiant lipophile choisi parmi les esters de dextrine et d'acide gras, de préférence le myristate de dextrine ;
- de 20 % à 30 % en poids d'au moins une charge choisie parmi les poudres d'acide aminé N-acylé ayant un groupe acyle en C8-C22 ; et
- de 60 % à 85 % en poids d'au moins une phase grasse contenant au moins 60 % en poids d'au moins une huile choisie parmi les huiles hydrocarbonées d'origine végétale, de préférence les triglycérides ;
les quantités en poids étant données par rapport au poids total de la composition.

Selon un quatrième mode de réalisation spécifique de l'invention, la composition anhydre comprend :
- de 3 % à 15 % en poids d'au moins un gélifiant lipophile choisi parmi les tri-esters d'acide gras et de mono ou polyglycérol tels que le tri-hydroxy-stéarate de glycéryle ;
- de 20 % à 50 % en poids d'au moins une charge choisie parmi les poudres d'acide aminé N-acylé ayant un groupe acyle en C8-C22 ; et
- de 40 % à 85 % en poids d'au moins une phase grasse contenant au moins 20 % en poids d'au moins une huile choisie parmi les huiles hydrocarbonées d'origine végétale, de préférence les triglycérides, les alcools de Guerbet, les esters d'acide gras en C8-C30 et d'alcool de Guerbet, les éthers d'alcools gras comportant de 6 à 20 atomes de carbone ;
les quantités en poids étant données par rapport au poids total de la composition.

Selon un mode de réalisation particulier, la composition anhydre comprend :
- de 3 % à 15 % en poids d'au moins un gélifiant lipophile choisi parmi les esters de dextrine et d'acide gras, de préférence le myristate de dextrine ;
- de 20 % à 50 % en poids, de préférence de 40 % à 50 % en poids d'au moins une charge choisie parmi les particules sphériques de cellulose ; et
- de 40 % à 85 % en poids d'au moins une phase grasse contenant au moins 25 % en poids d'au moins une huile choisie parmi les huiles hydrocarbonées d'origine végétale, de préférence les triglycérides ;
les quantités en poids étant données par rapport au poids total de la composition.

De préférence, la composition anhydre comprend :
- de 3 % à 15 % en poids d'au moins un gélifiant lipophile choisi parmi les esters de dextrine et d'acide gras, de préférence le myristate de dextrine ;
- de 40 % à 50 % en poids d'au moins une charge choisie parmi les particules sphériques de cellulose ; et
- de 40 % à 85 % en poids d'au moins une phase grasse contenant au moins 40 % en poids d'au moins une huile choisie parmi les huiles hydrocarbonées d'origine végétale, de préférence les triglycérides ;
les quantités en poids étant données par rapport au poids total de la composition.

Selon un sixième mode de réalisation spécifique de l'invention, la composition anhydre comprend :
- de 3 % à 15 % en poids d'au moins un gélifiant lipophile choisi parmi les tri-esters d'acide gras et de mono ou polyglycérol tels que le tri-hydroxy-stéarate de glycéryle ;
- de 20 % à 50 % en poids, de préférence de 30 % à 40 % en poids d'au moins une charge choisie parmi les particules sphériques de cellulose ; et
- de 40 % à 85 % en poids d'au moins une phase grasse contenant au moins 25 % en poids d'au moins une huile choisie parmi les huiles hydrocarbonées d'origine végétale, de préférence les triglycérides, les alcools de Guerbet, les esters d'acide gras en C8-C30 et d'alcool de Guerbet, les éthers d'alcools gras comportant de 6 à 20 atomes de carbone ;
les quantités en poids étant données par rapport au poids total de la composition.

Selon un mode de réalisation particulier, la composition anhydre comprend :
- de 3 % à 15 % en poids d'au moins un gélifiant lipophile choisi parmi les tri-esters d'acide gras et de mono ou polyglycérol tels que le tri-hydroxy-stéarate de glycéryle ;
- de 20 % à 50 % en poids, de préférence de 30 % à 40 % en poids d'au moins une charge choisie parmi les particules sphériques de cellulose ; et
- de 40 % à 85 % en poids d'au moins une phase grasse contenant au moins 25 % en poids d'au moins deux huiles dont l'une est choisie parmi les huiles hydrocarbonées d'origine végétale, de préférence les triglycérides ;
les quantités en poids étant données par rapport au poids total de la composition.

Selon un septième mode de réalisation spécifique de l'invention, la composition anhydre comprend :
- de 3 % à 15 % en poids d'au moins un gélifiant lipophile choisi parmi les esters de dextrine et d'acide gras, de préférence le palmitate de dextrine ;
- de 20 % à 50 % en poids, de préférence de 30 % à 40 % en poids d'au moins une charge choisie parmi les particules sphériques de cellulose ; et
- de 40 % à 85 % en poids d'au moins une phase grasse contenant au moins 25 % en poids d'au moins une huile choisie parmi les huiles hydrocarbonées d'origine végétale, de préférence les triglycérides, les alcools de Guerbet, les esters d'acide gras en C8-C30 et d'alcool de Guerbet, les éthers d'alcools gras comportant de 6 à 20 atomes de carbone ;
les quantités en poids étant données par rapport au poids total de la composition.

Selon un mode de réalisation particulier, la composition anhydre comprend :
- de 3 % à 15 % en poids d'au moins un gélifiant lipophile choisi parmi les esters de dextrine et d'acide gras, de préférence le palmitate de dextrine ;
- de 20 % à 50 % en poids, de préférence de 30 % à 40 % en poids d'au moins une charge choisie parmi les particules sphériques de cellulose ; et
- de 40 % à 85 % en poids d'au moins une phase grasse contenant au moins 25 % en poids d'au moins une huile choisie parmi les alcools de Guerbet et les esters d'acide gras en C8-C30 et d'alcool de Guerbet ;
les quantités en poids étant données par rapport au poids total de la composition.

De préférence, la composition anhydre comprend :
- de 3 % à 15 % en poids d'au moins un gélifiant lipophile choisi parmi les esters de dextrine et d'acide gras, de préférence le palmitate de dextrine ;
- de 30 % à 40 % en poids d'au moins une charge choisie parmi les particules sphériques de cellulose ; et
- de 40 % à 85 % en poids d'au moins une phase grasse contenant au moins 30 % en poids d'au moins une huile choisie parmi les alcools de Guerbet et les esters d'acide gras en C8-C30 et d'alcool de Guerbet ;
les quantités en poids étant données par rapport au poids total de la composition.

La composition selon l'invention peut en outre contenir un ou plusieurs polyols comprenant de 2 à 8 atomes de carbone. Par « polyols », il faut comprendre toute molécule organique comportant au moins deux groupements hydroxyle libres. Comme polyols, on peut citer par exemple la glycérine, les glycols comme le butylène glycol, le propylène glycol, l'isoprène glycol, le dipropylène glycol, l'hexylène glycol, les polyéthylènes glycols, le polypropylène glycol et notamment le dipropylène glycol, le propane-1,2-diol, le propane-1,3-diol. Selon un mode de réalisation particulier de l'invention, le polyol est choisi parmi la glycérine et le propane-1,3-diol. De préférence, le polyol est la glycérine.

On peut citer à titre d'exemple la glycerine commercialisée sous la dénomination GLYCERINE 4810 par la société OLEON ou encore sous la dénomination PALMERA G995V par la société KLK OLEO ou encore sous la dénomination REFINED GLYCERINE 99,5% PH. EURO par la société CARGILL.

On peut également citer le propane-1,3-diol commercialisé sous la dénomination ZEMEA PROPANEDIOL par la société Dupont Tate and Lyle Bio Products

On peut encore citer le butylène glycol commercialisé sous la dénomination 1,3 BUTYLENE GLYCOL par la société ALZO ou encore DAICEL.

On peut aussi citer le propylène glycol commercialisé sous la dénomination RADIANOL 4710 par la société OLEON

La quantité en polyols peut aller par exemple de 0 % à 20 % en poids, notamment de 0,1 à 20% en poids, de préférence de 3 % à 17 % en poids, mieux de 4 % à 15 % en poids, et encore mieux de 5 % à 10 % en poids par rapport au poids total de la composition.

La composition conforme à l'invention peut également comprendre un ou plusieurs alcools primaires, c'est-à-dire un alcool comportant de 1 à 6 atomes de carbone, tel que l'éthanol, le propanol, l'isopropanol, le butanol, le pentanol, l'hexanol et en particulier l'éthanol et l'isopropanol. Il s'agit de préférence de l'éthanol. L'ajout d'un tel alcool peut être notamment approprié lorsque la composition selon l'invention est utilisée comme produit pour le corps, le visage, ou les cheveux.

La quantité en alcools primaires peut aller par exemple de 0 % à 35 % en poids, notamment de 0,1 à 35% en poids, de préférence de 1 à 15 % en poids, et encore plus préférentiellement de 5 à 10 % en poids par rapport au poids total de la composition.

La composition de l'invention peut contenir un ou plusieurs des adjuvants habituels dans les domaines cosmétique et dermatologique tels que des tensioactifs ; des agents hydratants ; des actifs hydrophiles ou lipophiles ; des agents anti-radicaux libres ; des antioxydants ; des conservateurs ; des parfums ; des agents filmogènes ; des nacres ; des pigments ; des colorants ; et leurs mélanges.

La composition conforme à l'invention peut comprendre un ou plusieurs actifs.

On peut citer à titre d'exemple d'actif, et de façon non limitative, l'acide ascorbique et ses dérivés tels que le 5,6-di-O-diméthylsilylascorbate (notamment vendu par la Société Exsymol sous la référence PRO-AA), le sel de potassium du dl-alpha-tocopheryl-2l-ascorbyl-phosphate (notamment vendu par la Société Senju Pharmaceutical sous la référence SEPIVITAL EPC), l'ascorbyl phosphate de magnésium, l'ascorbyl phosphate de sodium (notamment vendu par la Société Roche sous la référence Stay-C 50) ; le phloroglucinol ; les enzymes ; et leurs mélanges. Parmi les actifs hydrophiles sensibles à l'oxydation, on utilise selon un mode de réalisation préféré de l'invention l'acide ascorbique. L'acide ascorbique peut être de toute nature. Ainsi, il peut être d'origine naturelle sous forme de poudre ou sous forme de jus d'orange de préférence concentré. Il peut être aussi d'origine synthétique, de préférence sous forme de poudre.

Comme autres actifs utilisables dans la composition de l'invention, on peut citer par exemple les agents hydratants tels que les hydrolysats de protéines ; les extraits naturels ; les anti-inflammatoires ; les oligomères procyannidoliques ; les vitamines comme la vitamine A (rétinol), la vitamine E (tocophérol) et autres anti-oxydants tel l'extrait de romarin, la vitamine B5 (panthénol), la vitamine B3 (niacinamide), les dérivés de ces vitamines (notamment esters) et leurs mélanges ; l'urée ; la caféine ; l'adenosine ; les dépigmentants tels que l'acide kojique, l'hydroquinone et l'acide caféique ; l'acide salicylique et ses dérivés ; les alpha-hydroxyacides tels que l'acide lactique et l'acide glycolique et leurs dérivés ; les rétinoïdes tels que les caroténoïdes et les dérivés de vitamine A ; l'hydrocortisone ; la mélatonine ; les extraits d'algues, de champignons, de végétaux, de levures, de bactéries ; les stéroïdes ; les actifs anti-bactériens ; les agents matifiants comme les fibres ; les agents tenseurs, les cires ; les filtres UV ; les huiles essentielles ; les céramides ; et leurs mélanges.

Les quantités de ces différents adjuvants et/ou actifs sont celles classiquement utilisées dans les domaines considérés. En particulier, ces quantités varient selon le but recherché et peuvent par exemple aller de 0,01 % à 20 %, et de préférence de 0,1 % à 10 % en poids du poids total de la composition.

Bien entendu, l'homme de l'art veillera à choisir le ou les éventuels adjuvants et/ou actifs ajoutés à la composition selon l'invention de manière telle que les propriétés avantageuses attachées intrinsèquement à la composition conforme à l'invention ne soient pas, ou substantiellement pas, altérées par l'addition envisagée.

La composition selon l'invention peut être utilisée pour toute application topique ; notamment, elle peut constituer une composition cosmétique ou dermatologique, de préférence une composition cosmétique, et en particulier dans le domaine cosmétique à efficacité perçue immédiate. Elle peut en particulier être utilisée pour le soin et/ou le démaquillage de la peau, des lèvres et /ou des yeux, et également comme composition pour le soin des cheveux. Elle peut également être utilisée comme déodorant ou comme produit solaire, ainsi que pour le nettoyage de la peau.

La composition conforme à l'invention peut notamment être utilisée comme produit à usage cosmétique pour le soin de la peau sur les axes anti-âge, peaux grasses, protection solaire, antiperspirants et déodorants, les produits cheveux et/ou cuir chevelu ainsi que styling, les produits parfumants ainsi que des produits de maquillage.

L'invention a aussi pour objet l'utilisation cosmétique d'une composition telle que définie ci-dessus, pour le soin, le démaquillage et/ou le nettoyage de la peau, des lèvres et/ou des yeux, et/ou pour le soin des cheveux.

La présente invention a encore pour objet un procédé cosmétique de démaquillage et/ou de soin de la peau, des lèvres et/ou des yeux, dans lequel on applique sur la peau, les lèvres et/ou les yeux, une composition telle que définie ci-dessus.

La présente invention a aussi pour objet un procédé cosmétique de soin des cheveux, dans lequel on applique sur les cheveux, une composition telle que définie ci-dessus.

La présente invention a également pour objet un procédé de traitement cosmétique de camouflage des imperfections colorielles et/ou des imperfections de relief de la peau.

Selon un mode préféré de réalisation de l'invention, la composition constitue une composition pour le soin de la peau du corps et / ou du visage, de préférence du visage.

La présente invention a également pour objet une composition aqueuse sous la forme d'une dispersion d'au moins une composition anhydre telle que définie précédemment dans une phase aqueuse.

La composition conforme à l'invention peut être obtenue de manière classique par l'homme du métier.

Selon un mode de réalisation particulier, la composition conforme à l'invention est obtenue selon le procédé suivant :
1) Mise en chauffe de la phase grasse jusqu'à obtenir une température de 70 °C à 80 °C suivant le cas ;
2) Ajout du gélifiant et gélification à 70 °C à 80 °C suivant le cas pendant 15 minutes sous émulseur, vitesse maximum adaptée au volume fabriqué ;
3) Refroidissement jusqu'à obtenir une température de 35 °C ;
4) Ajout des charges à 35 °C. Dispersion jusqu'à disparition des amas et obtention d'un jus homogène, lisse et mat d'aspect macroscopique.
5) Vidange de cuve.

Les exemples qui suivent permettront de mieux comprendre l'invention, sans toutefois présenter un caractère limitatif. Les matières premières sont nommées par leur nom chimique ou INCI. Les quantités indiquées sont en % en poids de matières premières, sauf mention contraire.

### Exemples

### Exemples 1 à 4

Les compositions 2 et 4 ne font pas partie de l'invention.

| **Composition** | **1** | **2** | **3** | **4** |
|---|---|---|---|---|
| SILICA (Silica Beads SB-300 de la société MYOSHI KASEI) | 35 | 35 | 35 | 35 |
| DEXTRIN PALMITATE (RHEOPEARL TL2-OR de CHIBA FLOUR MILLING) | 7,5 | 7,5 | 7 | 7 |
| CAPRYLIC/CAPRIC TRIGLYCERIDE (MYRITOL 318 de COGNIS (BASF) | - | 28,2 | 33,45 | 28,60 |
| OCTYLDODECYL MYRISTATE (MOD de GATTEFOSSE) | 33,45 | - | - | - |
| PRUNUS ARMENIACA KERNEL OIL (LIPOVOL P de LIPO CHEMICALS) | 10 | 10 | - | - |
| ISOPROPYL PALMITATE (Isopropyl palmitate de COGNIS) | 14 | 14,25 | 14 | 14,35 |
| UNDECANE (and) TRIDECANE (CETIOL UT de COGNIS) | - | 5 | - | 5 |
| Antioxydant | 0,05 | 0,05 | 0,05 | 0,05 |

### Procédé de préparation

La phase grasse est homogénéisée. Après introduction du gélifiant, phase de gélification une fois la température souhaitée atteinte (70 °C à 80 °C selon le cas).

Phase de refroidissement puis ajout de tout autre ingrédient de la formule puis des charges (température allant de 50 °C à 32 °C).

### Caractérisation des propriétés optiques

Les propriétés optiques des compositions 1 à 4 ont été caractérisées à l'aide de la mesure de Flou ou de Haze (effet voile ou calque) avec un appareil commercial de type « Hazemeter ».

Les mesures ont été effectuées selon le protocole suivant : sur un film transparent plastique (Byk), une couche de 25,4 µm d'épaisseur humide de la composition dont on cherche à évaluer le Flou est étalée, à l'aide d'un étaleur automatique. On laisse sécher pendant 1 heure à température ambiante puis on procède à la mesure de l'indice de flou (ou Haze) à l'aide d'un HAZE GARD de marque BYK GARDNER.

Les valeurs obtenues pour les compositions 1 à 10 sont les suivantes :

| **Composition** | **1** | **2** | **3** | **4** |
|---|---|---|---|---|
| Haze mesuré | 60 | 80 | 65,2 | 89,3 |

A titre indicatif, il est considéré qu'entre 20 et 60, on a obtenu un faible effet soft-focus, entre 60 et 80, on a obtenu un bon effet soft focus, et à partir de 90, on a obtenu un très bon effet soft-focus.

Ces mesures montrent donc que les compositions conformes à l'invention permettent d'obtenir un effet soft focus important.

### Evaluation sensorielle :

Les propriétés sensorielles des compositions 1 et 3 ont été évaluées après leur application sur la peau.

Les propriétés cosmétiques sont bonnes, notamment en termes de vitesse de pénétration, de non collant et de douceur.

### Stabilité

La stabilité des compositions a été évaluée sur une période de 2 mois à température ambiante (25 °C). Toutes les compositions sont stables après 2 mois de stockage à 25 °C.

### Exemples 5 à 9

Les compositions 7 et 9 ne font pas partie de l'invention.

| **Composition** | **5** | **6** | **7** |
|---|---|---|---|
| LAUROYL LYSINE (AMIHOPE LL de AJINOMOTO) | 35 | 35 | 35 |
| DEXTRIN MYRISTATE (RHEOPEARL MKL2 de CHIBA FLOUR MILLING) | - | 7 | - |
| TRI HYDROXYSTEARIN (THIXCIN R de ELEMENTIS) | 6,5 | - | 7,8 |
| CAPRYLIC/CAPRIC TRIGLYCERIDE (MYRITOL 318 de COGNIS (BASF) | - | - | 48,2 |
| DICAPRYLYL ETHER (CETIOL OE de COGNIS) | 30 | - | - |
| OCTYLDODECYL MYRISTATE (MOD de GATTEFOSSE) | - | 58 | - |
| ISOPROPYL PALMITATE (Isopropyl palmitate de COGNIS) | 28,5 | - | - |
| UNDECANE (and) TRIDECANE (CETIOL UT de COGNIS) | - | - | 9 |

| **Composition** | **8** | **9** |
|---|---|---|
| LAUROYL LYSINE (AMIHOPE LL de AJINOMOTO) | 25 | 20 |
| DEXTRIN MYRISTATE (RHEOPEARL MKL2 de CHIBA FLOUR MILLING) | 7,5 | - |
| TRI HYDROXYSTEARIN (THIXCIN R de ELEMENTIS) | - | 9,6 |
| CAPRYLIC/CAPRIC TRIGLYCERIDE (MYRITOL 318 de COGNIS (BASF) | 67,5 | - |
| DICAPRYLYL ETHER (CETIOL OE de COGNIS) | - | - |
| OCTYLDODECYL MYRISTATE (MOD de GATTEFOSSE) | - | - |
| ISOPROPYL PALMITATE (Isopropyl palmitate de COGNIS) | - | - |
| ISOPROPYL ISOSTEARATE ( RADIA 7739 d'OLEON) | - | 50,4 |
| UNDECANE (and) TRIDECANE (CETIOL UT de COGNIS) | - | 20 |

### Procédé de préparation

La phase grasse est homogénéisée. Après introduction du gélifiant, phase de gélification une fois la température souhaitée atteinte (70 °C à 80 °C selon le cas).

Phase de refroidissement puis ajout de tout autre ingrédient de la formule puis des charges (température allant de 50 °C à 32 °C).

### Caractérisation des propriétés optiques

Les propriétés optiques des compositions 5 à 9 ont été caractérisées à l'aide de la mesure de Flou ou de Haze (effet voile ou calque) avec un appareil commercial de type « Hazemeter ».

Les mesures ont été effectuées selon le protocole suivant : sur un film transparent plastique (Byk), une couche de 25,4 µm d'épaisseur humide de la composition dont on cherche à évaluer le Flou est étalée, à l'aide d'un étaleur automatique. On laisse sécher pendant 1 heure à température ambiante puis on procède à la mesure de l'indice de flou (ou Haze) à l'aide d'un HAZE GARD de marque BYK GARDNER.

Les valeurs obtenues pour les compositions 5 à 9 sont les suivantes :

| **Composition** | **5** | **6** | **7** | **8** | **9** |
|---|---|---|---|---|---|
| Haze mesuré | 85,1 | 79,1 | 90,5 | 74,23 | 79,4 |

A titre indicatif, il est considéré qu'entre 20 et 60, on a obtenu un faible effet soft-focus, entre 60 et 80, on a obtenu un bon effet soft focus, et à partir de 90, on a obtenu un très bon effet soft-focus.

Ces mesures montrent donc que les compositions conformes à l'invention permettent d'obtenir un effet soft focus important.

### Evaluation sensorielle :

Les propriétés sensorielles des compositions 5, 6 et 8 ont été évaluées après leur application sur la peau.

### Stabilité

La stabilité des compositions a été évaluée sur une période de 2 mois à température ambiante (25 °C). Les compositions sont stables après 2 mois de stockage à 25 °C.

### Exemples 10 et 11

Les compositions 10 et 11 suivantes ont été réalisées.

| **Composition** | **10** | **11** |
|---|---|---|
| CELLULOSE (CELLULOBEADS D-10 de DAITO KASEI KOGYO) | 50 | - |
| CELLULOSE (CELLULOBEADS D-5 de DAITO KASEI) | - | 35 |
| CAPRYLIC/CAPRIC TRIGLYCERIDE (MYRITOL 318 de COGNIS (BASF) | 45 | - |
| OCTYLDODECYL MYRISTATE (MOD de GATTEFOSSE) | - | 33,45 |
| ISOPROPYL PALMITATE (Isopropyl palmitate de COGNIS) | - | 14 |
| PRUNUS ARMENIACA KERNEL OIL (LIPOVOL P de LIPO CHEMICALS) | - | 10 |
| UNDECANE (and) TRIDECANE (CETIOL UT de COGNIS) | - | - |
| TRI HYDROXYSTEARIN (THIXCIN R de ELEMENTIS) | - | - |
| DEXTRIN PALMITATE (RHEOPEARL TL2-OR de CHIBA FLOUR MILLING) | - | 7,5 |
| DEXTRIN MYRISTATE (RHEOPEARL MKL2 de CHIBA FLOUR MILLING) | 5 | - |

### Procédé de préparation

La phase grasse est homogénéisée. Après introduction du gélifiant, phase de gélification une fois la température souhaitée atteinte (70 °C à 80 °C selon le cas).

Phase de refroidissement puis ajout de tout autre ingrédient de la formule puis des charges (température allant de 50 °C à 32 °C).

### Caractérisation des propriétés optiques

Les propriétés optiques des compositions 10 et 11 ont été caractérisées à l'aide de la mesure de Flou ou de Haze (effet voile ou calque) avec un appareil commercial de type « Hazemeter ».

Les mesures ont été effectuées selon le protocole suivant : sur un film transparent plastique (Byk), une couche de 25,4 µm d'épaisseur humide de la composition dont on cherche à évaluer le Flou est étalée, à l'aide d'un étaleur automatique. On laisse sécher pendant 1 heure à température ambiante puis on procède à la mesure de l'indice de flou (ou Haze) à l'aide d'un HAZE GARD de marque BYK GARDNER.

Les valeurs obtenues pour les compositions 10 et 11 sont les suivantes :

| **Composition** | **10** | **11** |
|---|---|---|
| Haze mesuré | 85,2 | 77 |

A titre indicatif, il est considéré qu'entre 20 et 60, on a obtenu un faible effet soft-focus, entre 60 et 80, on a obtenu un bon effet soft focus, et à partir de 90, on a obtenu un très bon effet soft-focus.

Ces mesures montrent donc que les compositions conformes à l'invention permettent d'obtenir un effet soft focus important.

### Evaluation sensorielle :

Les propriétés sensorielles des compositions 10 et 11 ont été évaluées après leur application sur la peau et sont résumées dans le tableau ci-dessous :

| Composition | Evaluation sensorielle |
|---|---|
| 10 | Fini peau très doux, filmogène non brillant. |
| 11 | Fini peau glissant poudré. |

### Stabilité

La stabilité des compositions 10 et 11 a été évaluée sur une période de 2 mois à température ambiante (25 °C). Toutes les compositions sont stables après 2 mois de stockage à 25 °C.

## Revendications

1. Composition anhydre comprenant :
- de 3 % à 15 % en poids d'au moins un gélifiant lipophile ;
- de 20 % à 50 % en poids d'au moins une charge choisie parmi les particules sphériques de silice poreuse, les particules sphériques de cellulose, et les poudres d'acide aminé N-acylé ayant un groupe acyle en C8-C22 ; et
- de 40 % à 85 % en poids d'au moins une phase grasse contenant au moins 20 % en poids, de préférence au moins 25 % en poids d'au moins une huile choisie parmi les huiles hydrocarbonées d'origine végétale, les alcools de Guerbet, les esters d'acide gras en C8-C30 et d'alcool de Guerbet, et les éthers d'alcools gras comportant de 6 à 20 atomes de carbone;
les quantités en poids étant données par rapport au poids total de la composition.

2. Composition selon la revendication 1 dans laquelle le ou les gélifiants lipophiles sont choisis parmi les esters de dextrine et d'acide gras et les tri-esters d'acide gras en C8-C30 et de mono ou polyglycérol avec 2 à 10 unités glycérol, de préférence les esters de dextrine et d'acide gras.

3. Composition selon l'une quelconque des revendications 1 à 2 dans laquelle le ou les esters de dextrine et d'acides gras sont choisis parmi les mono- ou poly-esters de dextrine et d'au moins un acide gras répondant à la formule (C) : 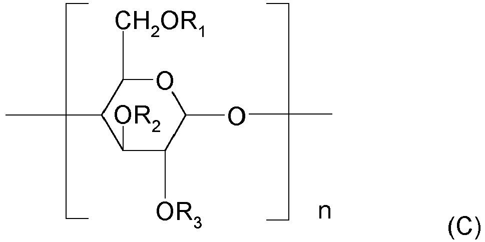 dans laquelle :
n est un entier allant de 3 à 150, notamment de 10 à 100, et de préférence de 15 à 40,
les radicaux R₁, R₂ et R₃, représentent un atome d'hydrogène ou un groupement acyle (R-CO-) dans lequel le radical R est un groupement hydrocarboné, linéaire ou ramifié, saturé ou insaturé, possédant 6 à 50, notamment 8 à 30, voire 12 à 22 et mieux 12 à 18 atomes de carbone, sous réserve qu'au moins un desdits radicaux R₁, R₂ ou R₃ est différent d'un atome d'hydrogène.

4. Composition selon la revendication 3 dans laquelle le ou les esters de dextrine et d'acides gras est un composé de formule (C) dans laquelle :
n varie avantageusement de 25 à 35, de préférence va de 27 à 33, et mieux est égal à 30,
le radical R-CO- est choisi parmi les radicaux caprylyle, caproyle, lauroyle, myristyle, palmityle, stéaryle, eicosanyle, docosanoyle, isovaléryle, éthyl-2 butyryle, éthylméthylacétyle, isoheptanyle, éthyl-2 hexanyle, isononanyle, isodécanyle, isotridécanyle, isomyristyle, isopalmityle, isostéaryle, isohexanyle, décènyle, dodécenyle, tétradécényle, myristyle, hexadécénoyle, palmitolyle, oléyle, élaidyle, eicosényle, sorbyle, linoléyle, linolényle, punicyle, arachidonyle, stéarolyle, et leurs mélanges, de préférence le radical R-CO- est le radical palmityle ou le radical myristyle, et encore plus préférentiellement le radical palmityle.

5. Composition selon l'une quelconque des revendications 2 à 4 dans laquelle l'acide gras du ou des tri-esters d'acide gras en C8-C30 et de mono ou polyglycérol avec 2 à 10 unités glycérol est un acide linéaire ou ramifié, saturé ou insaturé, comprenant de 10 à 24 atomes de carbone, encore plus préférentiellement de 12 à 22, et mieux de 16 à 20 atomes de carbone, substitué ou non substitué par un ou plusieurs groupements hydroxyle, de préférence un acide linéaire, saturé, substitué par au moins un groupement hydroxyle.

6. Composition selon l'une quelconque des revendications 2 à 5 dans laquelle le ou les tri-esters d'acide gras en C8-C30 et de mono ou polyglycérol sont choisis parmi les tri-esters d'acide gras et de monoglycérol, de préférence les tri(hydroxystéarate) de glycéryle, et encore plus préférentiellement le tris(12-hydroxystéarate) de glycéryle.

7. Composition selon l'une quelconque des revendications 1 à 6 dans laquelle la ou les charges sont choisies parmi les particules sphériques de silice poreuse.

8. Composition selon l'une quelconque des revendications 1 à 6 dans laquelle la ou les charges sont choisies parmi les particules sphériques de cellulose.

9. Composition selon l'une quelconque des revendications 1 à 6 dans laquelle la ou les charges sont choisies parmi les poudres d'acide aminé N-acylé ayant un groupe acyle en C8-C22.

10. Composition selon l'une quelconque des revendications 1 à 9 comprenant de 45 % à 85 % en poids d'au moins une phase grasse, de préférence de 45 % à 75 % en poids, et encore plus préférentiellement de 57 % à 70 % en poids du poids total de la composition.

11. Composition selon l'une quelconque des revendications 1 à 10 dans laquelle la phase grasse comprend au moins 25% en poids d'au moins une huile choisie parmi les huiles hydrocarbonées d'origine végétale, de préférence les triglycérides, les alcools de Guerbet, et les esters d'acide gras en C8-C30 et d'alcool de Guerbet.

12. Composition selon l'une quelconque des revendications 1 à 11 dans laquelle la phase grasse comprend au moins 25 % en poids, d'au moins deux huiles choisies parmi les huiles hydrocarbonées d'origine végétale, de préférence les triglycérides, les alcools de Guerbet, les esters d'acide gras en C8-C30 et d'alcool de Guerbet, et les alcanes linéaires en C7-C17.

13. Composition selon l'une quelconque des revendications 1 à 12 dans laquelle la ou les huiles hydrocarbonées d'origine végétale sont choisies parmi le squalane, les triglycérides liquides d'acides gras comportant de 4 à 30 atomes de carbone comme les triglycérides des acides heptanoïque ou octanoïque ou les huiles de jojoba, de babassu, de tournesol, d'olive, de noix de coco, de noix du brésil, de marula, de maïs, de soja, de courge, de pépins de raisin, de lin, de sésame, de noisette, d'abricot, de macadamia, d'arara, de coriandre, de ricin, d'avocat, les triglycérides des acides caprylique/caprique, l'huile de beurre de karité, de préférence les triglycérides des acides caprylique/caprique.

14. Composition selon l'une quelconque des revendications 12 à 13 dans laquelle le ou les alcanes linéaires en C7-C17, avantageusement d'origine végétale, comprennent de 9 à 15 atomes de carbone, et plus particulièrement de 11 à 13 atomes de carbone, de préférence sont choisis parmi le n-nonane (C₉), le n-décane (C₁₀), le n-undécane (C₁₁), le n-dodécane (C₁₂), le n-tridécane (C₁₃), le n-tétradecane (C₁₄), le n-pentadécane (C₁₅), le n-héxadécane (C₁₆) et le n-heptadécane (C₁₇) et leurs mélanges, et encore plus préférentiellement sont choisis parmi un mélange d'undécane (C₁₁) et de tridécane (C₁₃), le n-dodécane (C12) et le n-tétradécane (C14).

15. Composition selon l'une quelconque des revendications 1 à 14 dans laquelle les alcools de Guerbet et les esters d'acides gras en C8-C30 et d'alcools de Guerbet sont choisis parmi l'octyldodécanol et les esters d'octyldodécanol tels que le myristate d'octyldodécyle.

16. Composition selon l'une quelconque des revendications 1 à 10 dans laquelle les éthers d'alcools gras sont des éthers d'alcools gras comprenant de 8 à 12, et préférentiellement de 8 à 10 atomes de carbone, pouvant être obtenus à partir de deux alcools gras différents ou de deux alcools gras identiques, et de préférence à partir de deux alcools gras identiques tel que l'alcool caprylique (encore appelé octan-1-ol ou n-octanol).

17. Composition anhydre selon l'une quelconque des revendications 1 à 14 comprenant :
- de 3 % à 15 % en poids d'au moins un gélifiant lipophile choisi parmi les esters de dextrine et d'acide gras, de préférence le palmitate de dextrine ;
- de 30 % à 40 % en poids d'au moins une charge choisie parmi les particules sphériques de silice poreuse ; et
- de 40 % à 85 % en poids d'au moins une phase grasse contenant au moins 25 % en poids d'au moins une huile choisie parmi les huiles hydrocarbonées d'origine végétale, de préférence les triglycérides, et au moins 5 % en poids d'au moins une huile choisie parmi les alcanes linéaires en C7-C17 ;
les quantités en poids étant données par rapport au poids total de la composition.

18. Composition anhydre selon l'une quelconque des revendications 1 à 14 comprenant :
- de 3 % à 15 % en poids d'au moins un gélifiant lipophile choisi parmi les tri-esters d'acide gras en C8-C30 et de mono ou polyglycérol tels que le tri-hydroxy-stéarate de glycéryle ;
- de 30 % à 40 % en poids d'au moins une charge choisie parmi les poudres d'acide aminé N-acylé ayant un groupe acyle en C8-C22 ; et
- de 45 % à 85 % en poids d'au moins une phase grasse contenant au moins 40 % en poids d'au moins une huile choisie parmi les huiles hydrocarbonées d'origine végétale, de préférence les triglycérides, et au moins 5 % en poids d'au moins une huile choisie parmi les alcanes linéaires en C7-C17 ;
les quantités en poids étant données par rapport au poids total de la composition.

19. Composition anhydre selon l'une quelconque des revendications 1 à 13 comprenant :
- de 3 % à 15 % en poids d'au moins un gélifiant lipophile choisi parmi les esters de dextrine et d'acide gras, de préférence le myristate de dextrine ;
- de 40 % à 50 % en poids d'au moins une charge choisie parmi les particules sphériques de cellulose ; et
- de 40 % à 85 % en poids d'au moins une phase grasse contenant au moins 40 % en poids d'au moins une huile choisie parmi les huiles hydrocarbonées d'origine végétale, de préférence les triglycérides ;
les quantités en poids étant données par rapport au poids total de la composition.

20. Composition anhydre selon l'une quelconque des revendications 1 à 14 comprenant :
- de 3 % à 15 % en poids d'au moins un gélifiant lipophile choisi parmi les tri-esters d'acide gras en C8-C30 et de mono ou polyglycérol tels que le tri-hydroxy-stéarate de glycéryle ;
- de 30 % à 40 % en poids d'au moins une charge choisie parmi les particules sphériques de cellulose ; et
- de 45 % à 85 % en poids d'au moins une phase grasse contenant au moins 25 % en poids d'au moins une huile choisie parmi les huiles hydrocarbonées d'origine végétale, de préférence les triglycérides, et au moins 20 % en poids d'au moins une huile choisie parmi les alcanes linéaires en C7-C17 ;
les quantités en poids étant données par rapport au poids total de la composition.

21. Composition anhydre selon l'une quelconque des revendications 1 à 10 et 16 comprenant :
- de 3 % à 15 % en poids d'au moins un gélifiant lipophile choisi parmi les tri-esters d'acide gras en C8-C30 et de mono ou polyglycérol tels que le tri-hydroxy-stéarate de glycéryle ;
- de 30 % à 40 % en poids d'au moins une charge choisie parmi les poudres d'acide aminé N-acylé ayant un groupe acyle en C8-C22 ; et
- de 40 % à 85 % en poids d'au moins une phase grasse contenant au moins 25 % en poids d'au moins une huile choisie parmi les éthers d'alcools gras comportant de 6 à 20 atomes de carbone ;
les quantités en poids étant données par rapport au poids total de la composition.

22. Procédé de traitement cosmétique des matières kératiniques dans lequel on applique sur les matières kératiniques une composition telle que définie à l'une quelconque des revendications 1 à 21.

23. Procédé de traitement cosmétique de camouflage des imperfections colorielles et/ou des imperfections de reflief de la peau dans lequel on applique sur la peau une composition telle que définie à l'une quelconque des revendications 1 à 21.

24. Composition aqueuse sous la forme d'une dispersion d'au moins une composition anhydre telle que définie à l'une quelconque des revendications 1 à 21 dans une phase aqueuse.

## Patentansprüche

1. Wasserfreie Zusammensetzung, umfassend:
- 3 bis 15 Gew.-% mindestens eines lipophilen Geliermittels;
- 20 bis 50 Gew.-% mindestens eines Füllstoffs, der aus kugelförmigen Teilchen von porösem Siliciumdioxid, kugelförmigen Celluloseteilchen und Pulvern einer N-acetylierten Aminosäure mit einer C8-C22-Acylgruppe ausgewählt ist; und
- 40 bis 85 Gew.-% mindestens einer Fettphase, enthaltend mindestens 20 Gew.-%, vorzugsweise mindestens 25 Gew.-%, mindestens eines Öls, das aus Kohlenwasserstoffölen pflanzlicher Herkunft, Guerbet-Alkoholen, Estern von C8-C30-Fettsäure und Guerbet-Alkohol und Ethern von Fettalkoholen mit 6 bis 20 Kohlenstoffatomen ausgewählt ist;
wobei sich die Gewichtsmengen auf das Gesamtgewicht der Zusammensetzung beziehen.

2. Zusammensetzung nach Anspruch 1, wobei der lipophile Gelbildner bzw. die lipophilen Gelbildner aus Estern von Dextrin und Fettsäure und Triestern von C8-C30-Fettsäure und Mono- oder Polyglycerin mit 2 bis 10 Glycerineinheiten, vorzugsweise Estern von Dextrin und Fettsäure, ausgewählt ist bzw. sind.

3. Zusammensetzung nach einem der Ansprüche 1 bis 2, wobei der Ester bzw. die Ester von Dextrin und Fettsäuren aus Mono- oder Polyestern von Dextrin und mindestens einer Fettsäure der Formel (C) ausgewählt ist bzw. sind: 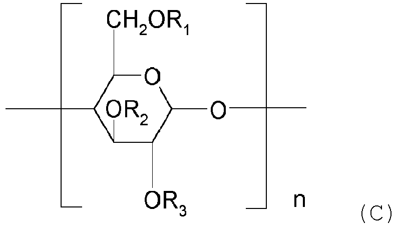 wobei:
n für eine ganze Zahl im Bereich von 3 bis 150, insbesondere von 10 bis 100 und vorzugsweise von 15 bis 40 steht,
die Reste R₁, R₂ und R₃ für ein Wasserstoffatom oder eine Acylgruppe (R-CO-) stehen, in der der Rest R für eine lineare oder verzweigte, gesättigte oder ungesättigte Kohlenwasserstoffgruppe mit 6 bis 50, insbesondere 8 bis 30, sogar 12 bis 22 und noch besser 12 bis 18 Kohlenstoffatomen steht, mit der Maßgabe, dass mindestens einer der Reste R₁, R₂ und R₃ von einem Wasserstoffatom verschieden ist.

4. Zusammensetzung nach Anspruch 3, wobei es sich bei dem Ester bzw. den Estern von Dextrin und Fettsäuren um eine Verbindung der Formel (C) handelt, wobei:
n vorteilhafterweise im Bereich von 25 bis 35 und vorzugsweise im Bereich von 27 bis 33 liegt und noch besser gleich 30 ist,
der Rest R-CO- aus Caprylyl-, Caproyl-, Lauroyl-, Myristyl-, Palmityl-, Stearyl-, Eicosanyl-, Docosanoyl-, Isovaleryl-, 2-Ethylbutyryl-, Ethylmethylacetyl-, Isoheptanyl-, 2-Ethylhexanyl-, Isononanyl-, Isodecanyl-, Isotridecanyl-, Isomyristyl-, Isopalmityl-, Isostearyl-, Isohexanyl-, Decenyl-, Dodecenyl-, Tetradecenyl-, Myristyl-, Hexadecenoyl-, Palmitolyl-, Oleyl-, Elaidyl-, Eicosenyl-, Sorbyl-, Linoleyl-, Linolenyl-, Punicyl-, Arachidonyl- und Steroylresten und Mischungen davon ausgewählt ist, wobei es sich bei dem Rest R-CO- vorzugsweise um den Palmitylrest oder den Myristylrest und noch weiter bevorzugt um den Palmitylrest handelt.

5. Zusammensetzung nach einem der Ansprüche 2 bis 4, wobei es sich bei der Fettsäure des Triesters bzw. der Triester von C8-C30-Fettsäure und Mono- oder Polyglycerin mit 2 bis 10 Glycerineinheiten um eine lineare oder verzweigte, gesättigte oder ungesättigte Säure mit 10 bis 24 Kohlenstoffatomen, vorzugsweise 12 bis 22 und noch besser 16 bis 20 Kohlenstoffatomen, die gegebenenfalls durch eine oder mehrere Hydroxylgruppen substituiert ist, vorzugsweise um eine lineare, gesättigte Säure, die durch mindestens eine Hydroxylgruppe substituiert ist, handelt.

6. Zusammensetzung nach einem der Ansprüche 2 bis 5, wobei der Triester bzw. die Triester von C8-C30-Fettsäure und Mono- oder Polyglycerin aus Triestern von Fettsäure und Monoglycerin, vorzugsweise Glyceryltri(hydroxystearat) und noch weiter bevorzugt Glyceryltris(12-hydroxystearat) ausgewählt ist bzw. sind.

7. Zusammensetzung nach einem der Ansprüche 1 bis 6, wobei der Füllstoff bzw. die Füllstoffe aus kugelförmigen Teilchen von porösem Siliciumdioxid ausgewählt ist bzw. sind.

8. Zusammensetzung nach einem der Ansprüche 1 bis 6, wobei der Füllstoff bzw. die Füllstoffe aus kugelförmigen Celluloseteilchen ausgewählt ist bzw. sind.

9. Zusammensetzung nach einem der Ansprüche 1 bis 6, wobei der Füllstoff bzw. die Füllstoffe aus Pulvern einer N-acetylierten Aminosäure mit einer C8-C22-Acylgruppe ausgewählt ist bzw. sind.

10. Zusammensetzung nach einem der Ansprüche 1 bis 9, umfassend 45 bis 85 Gew.-% mindestens einer Fettphase, vorzugsweise 45 bis 75 Gew.-% und noch weiter bevorzugt 57 bis 70 Gew.-%, bezogen auf das Gesamtgewicht der Zusammensetzung.

11. Zusammensetzung nach einem der Ansprüche 1 bis 10, wobei die Fettphase mindestens 25 Gew.-% mindestens eines Öls, das aus Kohlenwasserstoffölen pflanzlicher Herkunft, vorzugsweise Triglyceriden, Guerbet-Alkoholen und Estern von C8-C30-Fettsäure und Guerbet-Alkohol ausgewählt ist, umfasst.

12. Zusammensetzung nach einem der Ansprüche 1 bis 11, wobei die Fettphase mindestens 25 Gew.-% von mindestens zwei Ölen, die aus Kohlenwasserstoffölen pflanzlicher Herkunft, vorzugsweise Triglyceriden, Guerbet-Alkoholen, Estern von C8-C30-Fettsäure und Guerbet-Alkohol und linearen C7-C17-Alkanen ausgewählt ist, umfasst.

13. Zusammensetzung nach einem der Ansprüche 1 bis 12, wobei das Kohlenwasserstofföl bzw. die Kohlenwasserstofföle pflanzlicher Herkunft aus Squalan, flüssigen Triglyceriden von Fettsäuren mit 4 bis 30 Kohlenstoffatomen wie Triglyceriden von Heptan- oder Octansäure oder Jojobaöl, Babassuöl, Sonnenblumenöl, Olivenöl, Kokosnussöl, Paranussöl, Marulaöl, Maisöl, Sojaöl, Kürbisöl, Traubenkernöl, Leinöl, Sesamöl, Haselnussöl, Aprikosenöl, Macadamiaöl, Araraöl, Korianderöl, Ricinusöl, Avocadoöl, Capryl-/Caprinsäuretriglyceriden und Sheabutteröl, vorzugsweise Capryl-/Caprinsäuretriglyceriden, ausgewählt ist bzw. sind.

14. Zusammensetzung nach einem der Ansprüche 12 bis 13, wobei das lineare C7-C17-Alkan bzw. die linearen C7-C17-Alkane, vorzugsweise pflanzlicher Herkunft, 9 bis 15 Kohlenstoffatome und spezieller 11 bis 13 Kohlenstoffatome umfasst bzw. umfassen und vorzugsweise aus n-Nonan (C₉), n-Decan (C₁₀), n-Undecan (C₁₁), n-Dodecan (C₁₂), n-Tridecan (C₁₃), n-Tetradecan (C₁₄), n-Pentadecan (C₁₅), n-Hexadecan (C₁₆) und n-Heptadecan (C₁₇) und Mischungen davon ausgewählt ist bzw. sind und noch weiter bevorzugt aus einer Mischung von Undecan (C₁₁) und Tridecan (C₁₃), n-Dodecan (C₁₂) und n-Tetradecan (C₁₄) ausgewählt ist bzw. sind.

15. Zusammensetzung nach einem der Ansprüche 1 bis 14, wobei die Guerbet-Alkohole und Ester von C8-C30-Fettsäuren und Guerbet-Alkoholen aus Octyldodecanol und Estern von Octyldodecanol wie Octyldodecylmyristat ausgewählt sind.

16. Zusammensetzung nach einem der Ansprüche 1 bis 10, wobei es sich bei den Ethern von Fettalkoholen um Ether von Fettalkoholen mit 8 bis 12 und vorzugsweise 8 bis 10 Kohlenstoffatomen handelt, die aus zwei verschiedenen Fettalkoholen oder zwei identischen Fettalkoholen und vorzugsweise aus zwei identischen Fettalkoholen wie Caprylalkohol (auch bekannt als 1-Octanol oder n-Octanol) erhältlich sind.

17. Wasserfreie Zusammensetzung nach einem der Ansprüche 1 bis 14, umfassend:
- 3 bis 15 Gew.-% mindestens eines lipophilen Geliermittels, das aus Estern von Dextrin und Fettsäure, vorzugsweise Dextrinpalmitat, ausgewählt ist;
- 30 bis 40 Gew.-% mindestens eines Füllstoffs, der aus kugelförmigen Teilchen von porösem Siliciumdioxid ausgewählt ist; und
- 40 bis 85 Gew.-% mindestens einer Fettphase, enthaltend mindestens 25 Gew.-% mindestens eines Öls, das aus Kohlenwasserstoffölen pflanzlicher Herkunft, vorzugsweise Triglyceriden, ausgewählt ist, und mindestens 5 Gew.-% mindestens eines Öls, das aus linearen C7-C17-Alkanen ausgewählt ist;
wobei sich die Gewichtsmengen auf das Gesamtgewicht der Zusammensetzung beziehen.

18. Wasserfreie Zusammensetzung nach einem der Ansprüche 1 bis 14, umfassend:
- 3 bis 15 Gew.-% mindestens eines lipophilen Geliermittels, das aus Triestern von C8-C30-Fettsäure und Mono- oder Polyglycerin wie Glyceryltrihydroxystearat ausgewählt ist;
- 30 bis 40 Gew.-% mindestens eines Füllstoffs, der aus Pulvern einer N-acetylierten Aminosäure mit einer C8-C22-Acylgruppe ausgewählt ist; und
- 45 bis 85 Gew.-% mindestens einer Fettphase, enthaltend mindestens 40 Gew.-% mindestens eines Öls, das aus Kohlenwasserstoffölen pflanzlicher Herkunft, vorzugsweise Triglyceriden, ausgewählt ist, und mindestens 5 Gew.-% mindestens eines Öls, das aus linearen C7-C17-Alkanen ausgewählt ist;
wobei sich die Gewichtsmengen auf das Gesamtgewicht der Zusammensetzung beziehen.

19. Wasserfreie Zusammensetzung nach einem der Ansprüche 1 bis 13, umfassend:
- 3 bis 15 Gew.-% mindestens eines lipophilen Geliermittels, das aus Estern von Dextrin und Fettsäure, vorzugsweise Dextrinmyristat, ausgewählt ist;
- 40 bis 50 Gew.-% mindestens eines Füllstoffs, der aus kugelförmigen Celluloseteilchen ausgewählt ist; und
- 40 bis 85 Gew.-% mindestens einer Fettphase, enthaltend mindestens 40 Gew.-% mindestens eines Öls, das aus Kohlenwasserstoffölen pflanzlicher Herkunft, vorzugsweise Triglyceriden, ausgewählt ist;
wobei sich die Gewichtsmengen auf das Gesamtgewicht der Zusammensetzung beziehen.

20. Wasserfreie Zusammensetzung nach einem der Ansprüche 1 bis 14, umfassend:
- 3 bis 15 Gew.-% mindestens eines lipophilen Geliermittels, das aus Triestern von C8-C30-Fettsäure und Mono- oder Polyglycerin wie Glyceryltrihydroxystearat ausgewählt ist;
- 30 bis 40 Gew.-% mindestens eines Füllstoffs, der aus kugelförmigen Celluloseteilchen ausgewählt ist; und
- 45 bis 85 Gew.-% mindestens einer Fettphase, enthaltend mindestens 25 Gew.-% mindestens eines Öls, das aus Kohlenwasserstoffölen pflanzlicher Herkunft, vorzugsweise Triglyceriden, ausgewählt ist, und mindestens 20 Gew.-% mindestens eines Öls, das aus linearen C7-C17-Alkanen ausgewählt ist;
wobei sich die Gewichtsmengen auf das Gesamtgewicht der Zusammensetzung beziehen.

21. Wasserfreie Zusammensetzung nach einem der Ansprüche 1 bis 10 und 16, umfassend:
- 3 bis 15 Gew.-% mindestens eines lipophilen Geliermittels, das aus Triestern von C8-C30-Fettsäure und Mono- oder Polyglycerin wie Glyceryltrihydroxystearat ausgewählt ist;
- 30 bis 40 Gew.-% mindestens eines Füllstoffs, der aus Pulvern einer N-acetylierten Aminosäure mit einer C8-C22-Acylgruppe ausgewählt ist; und
- 40 bis 85 Gew.-% mindestens einer Fettphase, enthaltend mindestens 25 Gew.-% mindestens eines Öls, das aus Ethern von Fettalkoholen mit 6 bis 20 Kohlenstoffatomen ausgewählt ist;
wobei sich die Gewichtsmengen auf das Gesamtgewicht der Zusammensetzung beziehen.

22. Verfahren zur kosmetischen Behandlung von Keratinmaterialien, bei dem man auf die Keratinmaterialien eine Zusammensetzung gemäß einem der Ansprüche 1 bis 21 aufbringt.

23. Verfahren zur kosmetischen Behandlung zum Verbergen von farblichen Unvollkommenheiten der Haut und/oder Unvollkommenheiten des Hautreliefs, bei dem man auf die Haut eine Zusammensetzung gemäß einem der Ansprüche 1 bis 21 aufbringt.

24. Wässrige Zusammensetzung in Form einer Dispersion mindestens einer wasserfreien Zusammensetzung gemäß einem der Ansprüche 1 bis 21 in einer wässrigen Phase.

## Claims

1. Anhydrous composition comprising:
- from 3% to 15% by weight of at least one lipophilic gelling agent;
- from 20% to 50% by weight of at least one filler chosen from spherical porous silica particles, spherical cellulose particles and powders of an N-acylamino acid bearing a C8-C22 acyl group; and
- from 40% to 85% by weight of at least one fatty phase containing at least 20% by weight and preferably at least 25% by weight of at least one oil chosen from hydrocarbon-based oils of plant origin, Guerbet alcohols, esters of a C8-C30 fatty acid and of a Guerbet alcohol, and fatty alcohol ethers comprising from 6 to 20 carbon atoms;
the weight amounts being given relative to the total weight of the composition.

2. Compostion according to Claim 1, in which the lipophilic gelling agent(s) are chosen from fatty acid esters of dextrin and triesters of a C8-C30 fatty acid and of mono- or polyglycerol with 2 to 10 glycerol units, preferably fatty acid esters of dextrin.

3. Compostion according to either of Claims 1 and 2, in which the fatty acid esters of dextrin are chosen from mono- or polyesters of dextrin and of at least one fatty acid corresponding to formula (C): 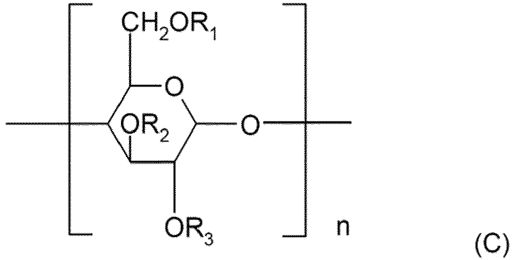 in which:
n is an integer ranging from 3 to 150, especially from 10 to 100 and preferably from 15 to 40;
the radicals R1, R2 and R3 represent a hydrogen atom or an acyl group (R-CO-) in which the radical R is a linear or branched, saturated or unsaturated hydrocarbon-based group containing from 6 to 50, especially from 8 to 30, or even 12 to 22 and better still 12 to 18 carbon atoms, with the proviso that at least one of said radicals R1, R2 or R3 is other than a hydrogen atom.

4. Compostion according to Claim 3, in which the fatty acid ester of dextrin is a compound of formula (C) in which:
n advantageously ranges from 25 to 35, preferably from 27 to 33, and better is equal to 30;
the radical R-CO- is chosen from caprylyl, caproyl, lauroyl, myristyl, palmityl, stearyl, eicosanyl, docosanoyl, isovaleryl, 2-ethylbutyryl, ethylmethylacetyl, isoheptanyl, 2-ethylhexanyl, isononanyl, isodecanyl, isotridecanyl, isomyristyl, isopalmityl, isostearyl, isohexanyl, decenyl, dodecenyl, tetradecenyl, myristyl, hexadecenoyl, palmitolyl, oleyl, elaidyl, eicosenyl, sorbyl, linoleyl, linolenyl, punicyl, arachidonyl and stearolyl radicals, and mixtures thereof; preferably, the radical R-CO- is the palmityl radical or the myristyl radical, and even more preferentially the palmityl radical.

5. Compostion according to any one of claims 2 to 4, in which the fatty acid of the triester(s) of a C8-C30 fatty acid and of mono- or polyglycerol with 2 to 10 glycerol units is a linear or branched, saturated or unsaturated acid, comprising from 10 to 24 carbon atoms, even more preferentially from 12 to 22 and better still from 16 to 20 carbon atoms, which is unsubstituted or substituted with one or more hydroxyl groups, preferably a saturated linear acid, substituted with at least one hydroxyl group.

6. Compostion according to any one of Claims 2 to 5, in which the triester(s) of a C8-C30 fatty acid and of mono- or polyglycerol are chosen from triesters of a fatty acid and of monoglycerol, preferably glyceryl tri(hydroxystearate), and even more preferentially glyceryl tris(12-hydroxystearate).

7. Compostion according to any one of Claims 1 to 6, in which the filler(s) are chosen from spherical porous silica particles.

8. Compostion according to any one of Claims 1 to 6, in which the filler(s) are chosen from spherical cellulose particles.

9. Compostion according to any one of Claims 1 to 6, in which the filler (s) are chosen from powders of an N-acylamino acid bearing a C8-C22 acyl group.

10. Compostion according to any one of Claims 1 to 9, comprising from 45% to 85% by weight of at least one fatty phase, preferably from 45% to 75% by weight and even more preferentially from 57% to 70% by weight relative to the total weight of the composition.

11. Compostion according to any one of Claims 1 to 10, in which the fatty phase comprises at least 25% by weight of at least one oil chosen from hydrocarbon-based oils of plant origin, preferably triglycerides, Guerbet alcohols, and esters of a C8-C30 fatty acid and of a Guerbet alcohol.

12. Compostion according to any one of Claims 1 to 11, in which the fatty phase comprises at least 25% by weight of at least two oils chosen from hydrocarbon-based oils of plant origin, preferably triglycerides, Guerbet alcohols, esters of a C8-C30 fatty acid and of a Guerbet alcohol, and linear C7-C17 alkanes.

13. Compostion according to any one of Claims 1 to 12, in which the hydrocarbon-based oil(s) of plant origin are chosen from squalane, liquid triglycerides of fatty acids comprising from 4 to 30 carbon atoms, for instance heptanoic or octanoic acid triglycerides, or jojoba oil, babassu oil, sunflower oil, olive oil, coconut oil, Brazil nut oil, marula oil, corn oil, soybean oil, marrow oil, grapeseed oil, linseed oil, sesame seed oil, hazelnut oil, apricot oil, macadamia nut oil, arara oil, coriander oil, castor oil, avocado oil, caprylic/capric acid triglycerides, and shea butter oil, preferably caprylic/capric acid triglycerides.

14. Compostion according to either of Claims 12 and 13, in which the linear C7-C17 alkane(s), advantageously of plant origin, comprise from 9 to 15 carbon atoms and more particularly from 11 to 13 carbon atoms, and are preferably chosen from n-nonane (C9), n-decane (C10), n-undecane (C11), n-dodecane (C12), n-tridecane (C13), n-tetradecane (C14), n-pentadecane (C15), n-hexadecane (C16) and n-heptadecane (C17) and mixtures thereof, and even more preferentially are chosen from a mixture of undecane (C11) and tridecane (C13), n-dodecane (C12) and n-tetradecane (C14).

15. Compostion according to any one of Claims 1 to 14, in which the Guerbet alcohols and the esters of C8-C30 fatty acids and of Guerbet alcohols are chosen from octyldodecanol and octyldodecanol esters such as octyldodecyl myristate.

16. Compostion according to any one of Claims 1 to 10, in which the fatty alcohol ethers are ethers of fatty alcohols comprising from 8 to 12 and preferentially from 8 to 10 carbon atoms, which may be obtained from two different fatty alcohols or from two identical fatty alcohols, and preferably from two identical fatty alcohols such as caprylyl alcohol (also known as 1-octanol or n-octanol).

17. Anhydrous composition according to any one of Claims 1 to 14, comprising:
- from 3% to 15% by weight of at least one lipophilic gelling agent chosen from fatty acid esters of dextrin, preferably dextrin palmitate;
- from 30% to 40% by weight of at least one filler chosen from porous spherical silica particles; and
- from 40% to 85% by weight of at least one fatty phase containing at least 25% by weight of at least one oil chosen from hydrocarbon-based oils of plant origin, preferably triglycerides, and at least 5% by weight of at least one oil chosen from linear C7-C17 alkanes;
the weight amounts being given relative to the total weight of the composition.

18. Anhydrous composition according to any one of Claims 1 to 14, comprising:
- from 3% to 15% by weight of at least one lipophilic gelling agent chosen from triesters of a C8-C30 fatty acid and of mono- or polyglycerol, such as glyceryl trihydroxystearate;
- from 30% to 40% by weight of at least one filler chosen from powders of an N-acylamino acid bearing a C8-C22 acyl group; and
- from 45% to 85% by weight of at least one fatty phase containing at least 40% by weight of at least one oil chosen from hydrocarbon-based oils of plant origin, preferably triglycerides, and at least 5% by weight of at least one oil chosen from linear C7-C17 alkanes;
the weight amounts being given relative to the total weight of the composition.

19. Anhydrous composition according to any one of Claims 1 to 13, comprising:
- from 3% to 15% by weight of at least one lipophilic gelling agent chosen from fatty acid esters of dextrin, preferably dextrin myristate;
- from 40% to 50% by weight of at least one filler chosen from spherical cellulose particles; and
- from 40% to 85% by weight of at least one fatty phase containing at least 40% by weight of at least one oil chosen from hydrocarbon-based oils of plant origin, preferably triglycerides;
the weight amounts being given relative to the total weight of the composition.

20. Anhydrous composition according to any one of Claims 1 to 14, comprising:
- from 3% to 15% by weight of at least one lipophilic gelling agent chosen from triesters of a C8-C30 fatty acid and of mono- or polyglycerol, such as glyceryl trihydroxystearate;
- from 30% to 40% by weight of at least one filler chosen from spherical cellulose particles; and
- from 45% to 85% by weight of at least one fatty phase containing at least 25% by weight of at least one oil chosen from hydrocarbon-based oils of plant origin, preferably triglycerides, and at least 20% by weight of at least one oil chosen from linear C7-C17 alkanes;
the weight amounts being given relative to the total weight of the composition.

21. Anhydrous composition according to any one of Claims 1 to 10 and 16, comprising:
- from 3% to 15% by weight of at least one lipophilic gelling agent chosen from triesters of a C8-C30 fatty acid and of mono- or polyglycerol, such as glyceryl trihydroxystearate;
- from 30% to 40% by weight of at least one filler chosen from powders of an N-acylamino acid bearing a C8-C22 acyl group; and
- from 40% to 85% by weight of at least one fatty phase containing at least 25% by weight of at least one oil chosen from fatty alcohol ethers comprising from 6 to 20 carbon atoms;
the weight amounts being given relative to the total weight of the composition.

22. Cosmetic process for treating keratin materials, in which a composition as defined in any one of Claims 1 to 21 is applied to the keratin materials.

23. Cosmetic treatment process for hiding skin color imperfections and/or skin relief imperfections, in which a composition as defined in any one of Claims 1 to 21 is applied to the skin.

24. Aqueous composition in the form of a dispersion of at least one anhydrous composition as defined in any one of Claims 1 to 21 in an aqueous phase.
